(19) Europäisches Patentamt
European Patent Office
Office européen des brevets

(11) EP 2 782 635 B1

# (12) EUROPEAN PATENT SPECIFICATION

(45) Date of publication and mention of the grant of the patent:
**02.09.2020 Bulletin 2020/36**

(21) Application number: **12795262.0**

(22) Date of filing: **19.11.2012**

(51) Int Cl.:
***A61N 1/05*** *(2006.01)* ***A61L 29/08*** *(2006.01)*
***A61L 29/14*** *(2006.01)*

(86) International application number:
**PCT/US2012/065896**

(87) International publication number:
**WO 2013/078139 (30.05.2013 Gazette 2013/22)**

## (54) FIBROUS MATRIX COATING MATERIALS

FASERMATRIXBESCHICHTUNGSMATERIALIEN

MATIÈRES DE REVÊTEMENT DE MATRICE FIBREUSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.11.2011 US 201161563218 P**

(43) Date of publication of application:
**01.10.2014 Bulletin 2014/40**

(73) Proprietor: **Cardiac Pacemakers, Inc.**
**St. Paul, Minnesota 55112 (US)**

(72) Inventors:
- **POLKINGHORNE, Jeannette C.**
  **Spring Lake Park, Minnesota 55432 (US)**
- **ARNHOLT, Devon N.**
  **Shoreview, Minnesota 55126 (US)**
- **ROHL, James P.**
  **Prescott, Minnesota 54021 (US)**
- **SCHMIDT, Brian L.**
  **White Bear Lake, Minnesota 55110 (US)**
- **SEPPALA, Jan**
  **Loretto, Minnesota 55357 (US)**
- **TADSEN, Richard L.**
  **Roseville, Minnesota 55357 (US)**
- **WILLOUGHBY, Patrick**
  **Hugo, Minnesota 55038 (US)**
- **ZHANG, Steve**
  **Blaine, Minnesota 55449 (US)**
- **DESAI, Shrojalkumar**
  **Lake Bluff, Minnesota 60044 (US)**
- **DELANEY, JR., Joseph T.**
  **Minneapolis, Minnesota 55418 (US)**
- **WULFMAN, David R.**
  **Minneapolis, Minnesota 55405 (US)**

(74) Representative: **Pfenning, Meinig & Partner mbB**
**Patent- und Rechtsanwälte**
**Joachimsthaler Straße 10-12**
**10719 Berlin (DE)**

(56) References cited:
**US-A1- 2007 051 531**
**US-A1- 2007 255 378**
**US-A1- 2009 105 796**
**US-A1- 2010 241 208**
**US-A1- 2011 054 581**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### TECHNICAL FIELD

The present invention relates to methods for manufacturing medical devices. More specifically, the invention relates to methods for coating medical devices and to coated medical devices.

### BACKGROUND

Cardiac pacing leads are well known and widely employed for carrying pulse stimulation signals to the heart from a battery operated pacemaker, or other pulse generating means, as well as for monitoring electrical activity of the heart from a location outside of the body. Electrical energy is applied to the heart via an electrode to return the heart to normal rhythm. Some factors that affect electrode performance include polarization at the electrode/tissue interface, electrode capacitance, sensing impedance, and voltage threshold. In all of these applications, it is highly desirable to optimize electrical performance characteristics at the electrode/tissue interface.

Recognized performance challenges of materials conventionally used as electrodes include difficulty controlling tissue in-growth, inflammation in the vicinity of the implanted device and/or the formation of fibrous scar tissue. These challenges may lead to difficulty in extracting the lead and/or reduced electrode performance over time.

Document US 2011/0054581 A1 discusses a medical electrical lead including an insulative lead body formed, at least in part, from a polyisobutylene urethane, urea or urethane/urea copolymer. Furthermore, a conductor may be disposed within the insulative lead body and extending from the proximal region to the distal region.

Document US 2007/0255378 A1 discusses the use of a fibrous matrix coating in combination with a medical lead.

### SUMMARY

Disclosed herein are various embodiments of a coated medical device, as well as methods for coating medical devices. The invention is defined by the appended claims.

While multiple embodiments are disclosed, still other embodiments of the present invention will become apparent to those skilled in the art from the following detailed description, which shows and describes illustrative embodiments of the invention. Accordingly, the drawings and detailed description are to be regarded as illustrative in nature and not restrictive.

### BRIEF DESCRIPTION OF THE DRAWINGS

Figure 1 is a schematic view of a medical electrical lead according to embodiments of the present invention.
Figure 2A and 2B are schematic longitudinal cross-sections of a medical electrical lead according to embodiments of the present invention.
Figure 3 is a schematic illustration of electrospinning.
Figure 4 is a schematic illustration of melt blowing.
Figure 5 is a graphical representation of experimental data.
Figure 6 is a graphical representation of experimental data.
Figure 7 is an image of a fibrous matrix on a coil of a lead.
Figure 8 is an image of a fibrous matrix.

While the invention is amenable to various modifications and alternative forms, specific embodiments have been shown by way of example in the drawings and are described in detail below. The intention, however, is not to limit the invention to the particular embodiments described. On the contrary, the invention is intended to cover all modifications, equivalents, and alternatives falling within the scope of the invention as defined by the appended claims.

### DETAILED DESCRIPTION

A more complete understanding of the present invention is available by reference to the following detailed description of numerous aspects and embodiments of the invention. The detailed description of the invention which follows is intended to illustrate but not limit the invention.

In accordance with various aspects of the invention, implantable and insertable medical devices are provided, which include one or more fibrous matrix containing one or more polyisobutylene urethane, urea or urethane/urea

copolymers (also referred to herein collectively as "polyisobutylene urethane copolymers").

Medical electrical devices of the present invention can typically include (a) an electronic signal generating component and (b) one or more leads. The electronic signal generating component commonly contains a source of electrical power (e.g., a sealed battery) and an electronic circuitry package, which produces electrical signals that are sent into the body (e.g., the heart, nervous system, etc.). Leads comprise at least one flexible elongated conductive member (e.g., a wire, cable, etc.), which is insulated along at least a portion of its length, generally by an elongated polymeric component often referred to as a lead body. The conductive member is adapted to place the electronic signal generating component of the device in electrical communication with one or more electrodes, which provide for electrical connection with the body. Leads are thus able to conduct electrical signals to the body from the electronic signal generating component. Leads may also relay signals from the body to the electronic signal generating component.

Examples of medical electrical devices of the present invention include, for example, implantable electrical stimulation systems including neurostimulation systems such as spinal cord stimulation (SCS) systems, deep brain stimulation (DBS) systems, peripheral nerve stimulation (PNS) systems, gastric nerve stimulation systems, cochlear implant systems, and retinal implant systems, among others, and cardiac systems including implantable cardiac rhythm management (CRM) systems, implantable cardioverter-defibrillators (ICD's), and cardiac resynchronization and defibrillation (CRDT) devices, among others.

Figure 1 is a partial cross-sectional view of a medical electrical lead 10, according to various embodiments of the present disclosure. According to some embodiments, the medical electrical lead 10 can be configured for implantation within a patient's heart. According to other embodiments, the medical electrical lead 10 is configured for implantation within a patient's neurovascular regions. In yet another embodiment, the lead 10 can be a lead for a cochlear implant. The medical electrical lead 10 includes an elongated, insulative lead body 12 extending from a proximal end 16 to a distal end 20. The proximal end 16 is configured to be operatively connected to a pulse generator via a connector 24. At least one conductor 32 extends from the connector 24 at the proximal end 16 of the lead 10 to one or more electrodes 28 at the distal end 20 of the lead 10. The conductor 32 can be a coiled or cable conductor. According to some embodiments where multiple conductors are employed, the lead can include a combination of coiled and cable conductors. When a coiled conductor is employed, according to some embodiments, the conductor can have either a co-radial or a co-axial configuration.

The lead body 12 is flexible, but substantially non-compressible along its length, and has a circular cross-section. According to certain embodiments, an outer diameter of the lead body 12 ranges from about 2 to about 15 French. In many embodiments, the lead body 12 does not include a drug collar or plug.

The medical electrical lead 10 can be unipolar, bipolar, or multi-polar depending upon the type of therapy to be delivered. In embodiments of the present disclosure employing multiple electrodes 28 and multiple conductors 32, each conductor 32 is adapted to be connected to an individual electrode 28 in a one-to-one manner allowing each electrode 28 to be individually addressable. Additionally, the lead body 12 can include one or more lumens adapted to receive a guiding element such as a guidewire or a stylet for delivery of the lead 10 to a target location within a patient's heart.

The electrodes 28 can have any electrode configuration as is known in the art. According to certain embodiments, at least one electrode can be a ring or partial ring electrode. According to another embodiment, at least one electrode 28 is a shocking coil. According to yet another embodiment of the present disclosure, at least one electrode 28 includes an exposed electrode portion and an insulated electrode portion. In some embodiments, a combination of electrode configurations can be used. The electrodes 28 can be coated with or formed from platinum, stainless steel, titanium, tantalum, palladium, MP35N, other similar conductive material, alloys of any of the foregoing including platinum-iridium alloys, and other combinations of the foregoing including clad metal layers or multiple metal materials.

According to various embodiments, the lead body 12 can include one or more fixation members for securing and stabilizing the lead body 12 including the one or more electrodes 28 at a target site within a patient's body. The fixation member(s) can be active or passive. An exemplary active fixation member includes a screw-in fixation member. Examples of passive fixation members can include pre-formed distal portions of the lead body 12 adapted to bear against vessel walls and/or expandable tines provided at the distal end of the lead body 12.

The lead 10 includes a fibrous matrix that is disposed over various parts of the insulative lead body 12. Figures 2A and 2B provide illustrative but non-limiting examples of regions of the lead 10 that may include a fibrous matrix. Figures 2A and 2B are schematic longitudinal cross-sectional views of the lead 10 of Figure 1, in which internal structure has been removed for clarity.

Figure 2A shows a fibrous matrix 40 disposed over a portion of the insulative lead body 12. The illustrated portion of the insulative lead body 12 may be adjacent an electrode such as the electrode 28, or it may be spaced apart from the electrodes. In contrast, Figure 2B illustrates a fibrous matrix 40 disposed over the electrode 28. While the fibrous matrix 40 is illustrated as covering all of the electrode 28, in some embodiments the fibrous matrix 40 covers only a small portion of the electrode 28, a substantial portion of the electrode 28, or an intervening fraction of the electrode 28.

In some embodiments, the fibrous matrix 40 may provide various beneficial functionalities to the lead 10. In

some embodiments, the fibrous matrix 40 may improve the abrasion resistance of the lead 10. In some embodiments, the fibrous matrix 40 may improve the electrical or thermal insulation of the lead 10. In some embodiments, the fibrous matrix 40 may provide improved control over tissue ingrowth, particularly at the site of the electrode 28. In certain embodiments, the amount of tissue ingrowth may be determined by tissue extraction in which the force required to remove an implanted lead 10 is measured with an Instron force gauge. In some embodiments, the thickness and average fiber diameter of fibrous matrix 40 impacts tissue ingrowth. The thickness and average fiber diameter of fibrous matrix 40 may also impact the ability to deliver electrophysiological therapy through fibrous matrix 40. In certain embodiments, the fibrous matrix 40 does not significantly impact the impedance of the lead 10.

The fibrous matrix 40 includes a plurality of randomly aligned fibers that comprise the matrix. In certain embodiments the fibrous matrix 40 may be formed by electrospinning or melt blowing, for example. The fibers may have diameters in the range of about 1 nanometer (nm) to 10,000 nm, for example. The fiber diameter size may be about 100 nm to 5,000 nm, for example. Suitable fiber diameter sizes also include about 40 nm to 2,000 nm, about 50 nm to 1,500 nm or about 100 nm to 1,000 nm, for example. In still further examples, the fiber diameter may be 1 nm to 800 nm, or 10 nm to 400 nm. In other examples, the average fiber diameter may be 400 nm to 10 microns or 800 nm to 10 microns. The fiber diameter size may be measured by taking the average size of the fibers. In certain embodiments, the fiber matrix may be formed partially or completely with hollow fibers using modified electrospinning and meltblowing techniques. The fiber size may affect tissue and growth. For example, the fibrous matrix 40 having an average diameter size greater than 800 nm may experience tissue ingrowth. In certain embodiments in which tissue ingrowth is not desired, the fibrous matrix 40 may have an average fiber diameter of less than about 800 nm, or less than about 400 nm.

The fibrous matrix 40 may have an average fiber-to fiber-spacing in the range of about 10 nm to about 100 microns, about 1 micron to about 100 microns, about 10 microns to about 50 microns, or about 10 microns to about 25 microns. In some embodiments, fiber-to-fiber spacing may be measured with a scanning microscope. In some embodiments, the fiber spacing between adjacent fibers may be adjusted or regulated to control tissue ingrowth while minimizing impact on pacing capability. This can be accomplished, for example, by altering the deposition parameters or deposition material. In some embodiments where tissue ingrowth may not be desired, the fibrous matrix 40 may have a smaller fiber-to-fiber spacing. For example, the fibrous matrix 40 may have a fiber-to-fiber spacing of about 10 nm to about 50 microns, about 50 nm to about 25 microns, or about 75 nm to about 1 micron. In other embodiments where tissue ingrowth is desired, the fibrous matrix 40 may have a larger fiber-to-fiber spacing. For example, the fibrous matrix 40 may have a fiber-to-fiber spacing of about 10 microns to about 100 microns, about 10 microns to about 50 microns, or about 10 microns to about 25 microns.

Various thicknesses can be obtained by varying the process conditions. For example during electrospinning, various thicknesses can be obtained by varying the flow rate, number of cycles, and rotational speed of the element to be coated. Suitable thicknesses for the fibrous matrix may be 1 nm to 1000 microns, 1 nm to 100 microns, 10 nm to 10 microns, or 70 nm to 250 nm. In some embodiments, tissue in-growth can be controlled by the thickness of the fibrous matrix 40.

Pores are formed between fibers of the fibrous matrix 40. In some embodiments, the fibrous matrix 40 can have an average pore size of 1 nm to 100 microns. In other embodiments, the fibrous matrix 40 can have an average pore size between 10 nanometers and 10 microns. As discussed herein, the pore size may inhibit or promote tissue ingrowth in the fibrous matrix 40. In certain embodiments, the fibrous matrix 40 may inhibit tissue ingrowth and may have an average pore size of 10 nm to 800 nm, 10 nm to 600 nm, or 10 nm to 400 nm. In other embodiments, the fibrous matrix 40 may promote tissue ingrowth in the fibrous matrix 40 and may have an average pore size of 400 nm to 100 microns, 400 nm to 10 microns, or 500 nm to 10 microns.

The spacing between fibers of the fibrous matrix 40 provides a porosity. In some embodiments, the porosity can be adjusted or regulated to control fiber ingrowth and/or conductivity. For example during electrospinning, various porosities may be obtained by varying the flow rate, number of cycles and the element to be coated. In one example, porosity can be measured using a Gurley Tester. In some embodiments, the fibrous matrix 40 has a porosity of at least 40%, 60%, 75% or 85%.

In some embodiments, particularly when the fibrous matrix 40 is disposed at least partially over an electrode such as the electrode 28, the fibrous matrix 40 may have sufficient fiber spacing to permit ions to flow through the fibrous matrix 40 such that electrical contact may be made with the electrode 28.

A wide range of polymers may be used to prepare the fibrous matrix 40, including both conductive and non-conductive polymer materials. Suitable non-conductive polymers (i.e. polymers that are not intrinsically conductive) include homopolymers, copolymers and terpolymers of various polyurethanes (such as polyether, polyester and polyisobutylene (PIB) polyurethanes). The non-conductive material in certain embodiments is free or substantially free of dopant materials that facilitate polymer conductivity. In another embodiment, the conductive material may comprises less than 5 weight percent (wt%) dopant, more particularly, less than 1 wt% dopant, even more particularly less than 0.5 wt% dopant.

In certain embodiments, the fibrous matrix 40 is formed from one or more polyisobutylene urethane, urea or

urethane/urea copolymers (also referred to herein collectively as "polyisobutylene urethane copolymers"). Examples of such copolymers and methods for their synthesis are generally described in WO 2008/060333, WO 2008/066914, U.S. Application No. 12/492,483 filed on June 26, 2009, entitled "Polyisobutylene Urethane, Urea and Urethane/Urea Copolymers and Medical Devices Containing the Same," and U.S. Application No. 12/874,887, filed September 2, 2010, and entitled "Medical Devices Including Polyisobutylene Based Polymers and Derivatives Thereof", all of which are incorporated herein by reference in their entirety.

As is well known, "polymers" are molecules containing multiple copies (e.g., from 5 to 10 to 25 to 50 to 100 to 250 to 500 to 1000 or more copies) of one or more constitutional units, commonly referred to as monomers. As used herein, the term "monomers" may refer to free monomers and to those that have been incorporated into polymers, with the distinction being clear from the context in which the term is used.

Polymers may take on a number of configurations including linear, cyclic and branched configurations, among others. Branched configurations include star-shaped configurations (e.g., configurations in which three or more chains emanate from a single branch point), comb configurations (e.g., configurations having a main chain and a plurality of side chains, also referred to as "graft" configurations), dendritic configurations (e.g., arborescent and hyperbranched polymers), and so forth.

As used herein, "homopolymers" are polymers that contain multiple copies of a single constitutional unit (i.e., a monomer). "Copolymers" are polymers that contain multiple copies of at least two dissimilar constitutional units.

Polyurethanes are a family of copolymers that are synthesized from polyfunctional isocyanates (e.g., diisocyanates, including both aliphatic and aromatic diisocyanates) and polyols (e.g., macroglycols). Commonly employed macroglycols include polyester diols, polyether diols and polycarbonate diols that form polymeric segments of the polyurethane. Typically, aliphatic or aromatic diols or diamines are also employed as chain extenders, for example, to impart improved physical properties to the polyurethane. Where diamines are employed as chain extenders, urea linkages are formed and the resulting polymers may be referred to as polyurethane/polyureas.

Polyureas are a family of copolymers that are synthesized from polyfunctional isocyanates and polyamines, for example, diamines such as polyester diamines, polyether diamines, polysiloxane diamines, polyhydrocarbon diamines and polycarbonate diamines. As with polyurethanes, aliphatic or aromatic diols or diamines may be employed as chain extenders.

According to certain aspects of the invention, the polyisobutylene urethane copolymer includes (a) one or more polyisobutylene segments, (b) one or more additional polymeric segments (other than polyisobutylene segments), (c) one or more segments that includes one or more diisocyanate residues, and optionally (d) one or more chain extenders.

As used herein, a "polymeric segment" or "segment" is a portion of a polymer. Segments can be unbranched or branched. Segments can contain a single type of constitutional unit (also referred to herein as "homopolymeric segments") or multiple types of constitutional units (also referred to herein as "copolymeric segments") which may be present, for example, in a random, statistical, gradient, or periodic (e.g., alternating) distribution.

The polyisobutylene segments of the polyisobutylene urethane copolymers are generally considered to constitute soft segments, while the segments containing the diisocyanate residues are generally considered to constitute hard segments. The additional polymeric segments may include soft or hard polymeric segments. As used herein, soft and hard segments are relative terms to describe the properties of polymer materials containing such segments. Without limiting the foregoing, a soft segment may display a Tg that is below body temperature, more typically from 35 °C to 20 °C to 0 °C to -25 °C to -50 °C or below. A hard segment may display a Tg that is above body temperature, more typically from 40 °C to 50 °C to 75 °C to 100 °C or above. Tg can be measured by differential scanning calorimetry (DSC), dynamic mechanical analysis (DMA) and thermomechanical analysis (TMA).

In certain embodiments, the soft segment is present in the amount of 45, 50, 55, 60, 65, 70, 75, 80, 85 or 90 wt%. In other embodiments, the soft segment is present in the amount of 40 to 90 wt% and the hard segment is present in the amount of 10 to 60 wt%.

Suitable soft segments include linear, branched or cyclic polyalkyl, polyalkene and polyalkenyl segments, polyether segments, fluoropolymer segments including fluorinated polyether segments, polyester segments, poly(acrylate) segments, poly(methacrylate) segments, polysiloxane segments and polycarbonate segments.

Examples of suitable polyether segments include linear, branched and cyclic homopoly(alkylene oxide) and copoly(alkylene oxide) segments, including homopolymeric and copolymeric segments formed from one or more, among others, methylene oxide, dimethylene oxide (ethylene oxide), trimethylene oxide, propylene oxide, tetramethylene oxide, pentamethylene oxide, hexamethylene oxide, octamethylene oxide and decamethylene oxide.

Examples of suitable fluoropolymer segments include perfluoroacrylate segments and fluorinated polyether segments, for example, linear, branched and cyclic homopoly(fluorinated alkylene oxide) and copoly(fluorinated alkylene oxide) segments, including homopolymeric and copolymeric segments formed from one or more of, among others, perfluoromethylene oxide, perfluorodimethylene oxide (perfluoroethylene oxide), perfluorotrimethylene oxide and perfluoropropylene oxide.

Examples of suitable polyester segments include linear, branched and cyclic homopolymeric and copolymeric

segments formed from one or more of, among others, alkyleneadipates including ethyleneadipate, propyleneadipate, tetramethyleneadipate, and hexamethyleneadipate.

Examples of suitable poly(acrylate) segments include linear, branched and cyclic homopoly(acrylate) and copoly(acrylate) segments, including homopolymeric and copolymeric segments formed from one or more of, among others, alkyl acrylates such as methyl acrylate, ethyl acrylate, propyl acrylate, isopropyl acrylate, butyl acrylate, sec-butyl acrylate, isobutyl acrylate, 2-ethylhexyl acrylate and dodecyl acrylate.

Examples of suitable poly(methacrylate) segments include linear, branched and cyclic homopoly(methacrylate) and copoly(methacrylate) segments, including homopolymeric and copolymeric segments formed from one or more of, among others, alkyl methacryates such as hexyl methacrylate, 2-ethylhexyl methacrylate, octyl methacrylate, dodecyl methacrylate and octadecyl methacrylate.

Examples of suitable polysiloxane segments include linear, branched and cyclic homopolysiloxane and copolysiloxane segments, including homopolymeric and copolymeric segments formed from one or more of, among others, dimethyl siloxane, diethyl siloxane, and methylethyl siloxane.

Examples of suitable polycarbonate segments include those comprising one or more types of carbonate units,

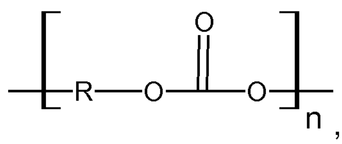

where R may be selected from linear, branched and cyclic alkyl groups. Specific examples include homopolymeric and copolymeric segments formed from one or more of, among others, ethylene carbonate, propylene carbonate, and hexamethylene carbonate.

Examples of hard polymeric segments include various poly(vinyl aromatic) segments, poly(alkyl acrylate) and poly(alkyl methacrylate) segments.

Examples of suitable poly(vinyl aromatic) segments include linear, branched and cyclic homopoly(vinyl aromatic) and copoly(vinyl aromatic) segments, including homopolymeric and copolymeric segments formed from one or more vinyl aromatic monomers including, among others, styrene, 2-vinyl naphthalene, alphamethyl styrene, p-methoxystyrene, p-acetoxystyrene, 2-methylstyrene, 3-methylstyrene and 4-methylstyrene.

Examples of suitable poly(alkyl acrylate) segments include linear, branched and cyclic homopoly(alkyl acrylate) and copoly(alkyl acrylate) segments, including homopolymeric and copolymeric segments formed from one or more acrylate monomers including, among others, tert-butyl acrylate, hexyl acrylate and isobornyl acrylate.

Examples of suitable poly(alkyl methacrylate) segments include linear, branched and cyclic homopoly(alkyl methacrylate) and copoly(alkyl methacrylate) segments, including homopolymeric and copolymeric segments formed from one or more alkyl methacrylate monomers including, among others, methyl methacrylate, ethyl methacrylate, isopropyl methacrylate, isobutyl methacrylate, t-butyl methacrylate, and cyclohexyl methacrylate.

Particularly suitable polyisobutylene urethane copolymers include (a) a polyisobutylene soft segment, (b) a polyether soft segment, (c) a hard segment containing diisocyanate residues, (d) optional chain extenders as further described below and/or (e) optional end capping materials as further described below.

The weight ratio of soft segments to hard segments in the polyisobutylene urethane copolymers of the present invention can be varied to achieve a wide range of physical and mechanical properties, including Shore Hardness, and to achieve an array of desirable functional performance. For example, the weight ratio of soft segments to hard segments in the polymer can be varied from 99:1 to 95:5 to 90:10 to 75:25 to 50:50 to 25:75 to 10:90 to 5:95 to 1:99, more particularly from 95:5 to 90:10 to 80:20 to 70:30 to 65:35 to 60:40 to 50:50, and even more particularly, from about 80:20 to about 50:50.

The Shore Hardness of the polyisobutylene urethane copolymers of embodiments of the present invention can be varied by controlling the weight ratio of soft segments to hard segments. Suitable Shore Hardness ranges include from 45A to 70D. Additional suitable Shore Hardness ranges include for example, from 45A, and more particularly from 50A to 52.5A to 55A to 57.5A to 60A to 62.5A to 65A to 67.5A to 70A to 72.5A to 75A to 77.5A to 80A to 82.5A to 85A to 87.5A to 90A to 92.5A to 95A to 97.5A to 100A. In certain embodiments, a polyisobutylene urethane copolymer with a soft segment to hard segment weight ratio of 80:20 results in a Shore Hardness of about 60-71A, a polyisobutylene urethane copolymer having a soft segment to hard segment weight ratio of 65:35 results in a Shore Hardness of 80-83A, a polyisobutylene urethane copolymer having a soft segment to hard segment weight ratio of 60:40 result in a Shore Hardness 95-99A, and a polyisobutylene urethane copolymer having a soft segment to hard segment weight ratio of 50:50 result in a Shore Hardness >100A. Higher hardness materials (e.g., 55 D and above up to 75 D) can also be prepared by increasing the ratio of hard to soft segments. Such harder materials may be particularly suitable for use in the PG header device, tip and pin areas of leads and headers of neuromodulation cans.

The polyisobutylene and additional polymeric segments can vary widely in molecular weight, but typically are

composed of between 2 and 100 repeat units (monomer units), among other values, and can be incorporated into the polyisobutylene polyurethane copolymers of the invention in the form of polyol (e.g., diols, triols, etc.) or polyamine (e.g., diamines, triamines, etc.) starting materials. Although the discussion to follow is generally based on the use of polyols, analogous methods may be performed and analogous compositions may be created using polyamines and polyol/polyamine combinations.

Suitable polyisobutylene polyol starting materials include linear polyisobutylene diols and branched (three-arm) polyisobutylene triols. More specific examples include linear polyisobutylene diols with a terminal -OH functional group at each end. Further examples of polyisobutylene polyols include poly(styrene-co-isobutylene) diols and poly(styrene-b-isobutylene-b-styrene) diols which may be formed, for example, using methods analogous to those described in See, e.g., J.P. Kennedy et al., "Designed Polymers by Carbocationic Macromolecular Engineering: Theory and Practice," Hanser Publishers 1991, pp. 191-193, Joseph P. Kennedy, Journal of Elastomers and Plastics 1985 17: 82-88, and the references cited therein. The polyisobutylene diol starting materials can be formed from a variety of initiators as known in the art. In certain embodiments, the polyisobutylene diol starting material is a saturated polyisobutylene diol that is devoid of C=C bonds.

Examples of suitable polyether polyol starting materials include polytetramethylene oxide diols and polyhexamethylene diols, which are available from various sources including Sigma-Aldrich Co., Saint Louis, MO, USA and E. I. duPont de Nemours and Co., Wilmington, DE, USA. Examples of polysiloxane polyol starting materials include polydimethylsiloxane diols, available from various sources including Dow Corning Corp., Midland MI, USA, Chisso Corp., Tokyo, Japan. Examples of suitable polycarbonate polyol starting materials include polyhexamethylene carbonate diols such as those available from Sigma-Aldrich Co.. Examples of polyfluoroalkylene oxide diol starting materials include ZDOLTX, Ausimont, Bussi, Italy, a copolyperfluoroalkylene oxide diol containing a random distribution of $-CF_2CF_2O-$ and $-CF_2O-$ units, end-capped by ethoxylated units, i.e., $H(OCH_2CH_2)_nOCH_2CF_2O(CF_2CF_2O)_p(CF_2O)_qCF_2CH_2O(CH_2CH_2O)_nH$, where n, p and q are integers. Suitable polystyrene diol starting materials ($\alpha,\omega$-dihydroxy-terminated polystyrene) of varying molecular weight are available from Polymer Source, Inc., Montreal, Canada. Polystyrene diols and three-arm triols may be formed, for example, using procedures analogous to those described in M. Weißmüller et al., "Preparation and end-linking of hydroxyl-terminated polystyrene star macromolecules," Macromolecular Chemistry and Physics 200(3), 1999, 541-551.

In some embodiments, polyols (e.g., diols, triols, etc.) are synthesized as block copolymer polyols. Examples of such block copolymer polyols include poly(tetramethylene oxide-*b*-isobutylene) diol, poly(tetramethylene oxide-*b*-isobutylene-*b*-tetramethylene oxide) diol, poly(dimethyl siloxane-*b*-isobutylene) diol, poly(dimethyl siloxane-*b*-isobutylene-*b*-dimethyl siloxane) diol, poly(hexamethylene carbonate-*b*-isobutylene) diol, poly(hexamethylene carbonate-*b*-isobutylene-*b*-hexamethylene carbonate) diol, poly(methyl methacrylate-*b*-isobutylene) diol, poly(methyl methacrylate-*b*-isobutylene-*b*-methyl methacrylate) diol, poly(styrene-*b*-isobutylene) diol and poly(styrene-*b*-isobutylene-*b*-styrene) diol (SIBS diol).

Diisocyanates for use in forming the urethane copolymers of the invention include aromatic and non-aromatic (e.g., aliphatic) diisocyanates. Aromatic diisocyanates may be selected from suitable members of the following, among others: 4,4'-methylenediphenyl diisocyanate (MDI), 2,4- and/or 2,6-toluene diisocyanate (TDI), 1,5-naphthalene diisocyanate (NDI), para-phenylene diisocyanate, 3,3'-tolidene-4,4'-diisocyanate and 3,3'-dimethyl-diphenylmethane-4,4'-diisocyanate. Non-aromatic diisocyanates may be selected from suitable members of the following, among others: 1,6-hexamethylene diisocyanate (HDI), 4,4'-dicyclohexylmethane diisocyanate, 3-isocyanatomethyl-3,5,5-trimethylcyclohexyl isocyanate (isophorone diisocyanate or IPDI), cyclohexyl diisocyanate, and 2,2,4-trimethyl-1,6-hexamethylene diisocyanate (TMDI).

In a particular embodiment, a polyether diol such as polytetramethylene oxide diol (PTMO diol), polyhexametheylene oxide diol (PHMO diol), polyoctamethylene oxide diol or polydecamethylene oxide diol is combined with the polyisobutylene diol and diisocyanate to form a polyisobutylene polyurethane copolymer with generally uniform distribution of the polyurethane hard segments, polyisobutylene segments and polyether segments to achieve favorable micro-phase separation in the polymer. The polyether segments may also improve key mechanical properties such as Shore Hardness, tensile strength, tensile modulus, flexural modulus, elongation, tear strength, flex fatigue, tensile creep, and abrasion performance, among others.

The polyisobutylene urethane copolymers in accordance with the invention may further include one or more optional chain extender residues and/or end groups. Chain extenders can increase the hard segment length (or, stated another way, can increase the ratio of hard segment material to soft segment material in the urethane, urea or urethane/urea polymer), which can in turn result in a polymer with higher modulus, lower elongation at break and increased strength. For instance the molar ratio of soft segment to chain extender to diisocyanate (SS:CE:DI) can range, for example, from 1:9:10 to 2:8:10 to 3:7:10 to 4:6:10 to 5:5:10 to 6:4:10 to 7:3:10 to 8:2:10 to 9:1:10.

Chain extenders are typically formed from aliphatic or aromatic diols (in which case a urethane bond is formed upon reaction with an isocyanate group) or aliphatic or aromatic diamines (in which case a urea bond is formed upon reaction with an isocyanate group). Chain extenders may be selected from suitable members of the following, among

others: alpha,omega-alkane diols such as ethylene glycol (1,2-ethane diol), 1,4-butanediol, 1,6-hexanediol, alpha,omega-alkane diamines such as ethylene diamine, dibutylamine (1,4-butane diamine) and 1,6-hexanediamine, or 4,4'-methylene bis(2-chloroaniline). Chain extenders may be also selected from suitable members of, among others, short chain diol polymers (e.g., alpha,omega-dihydroxy-terminated polymers having a molecular weight less than or equal to 1000) based on hard and soft polymeric segments (more typically soft polymeric segments) such as those described above, including short chain polyisobutylene diols, short chain polyether polyols such as polytetramethylene oxide diols, short chain polysiloxane diols such as polydimethylsiloxane diols, short chain polycarbonate diols such as polyhexamethylene carbonate diols, short chain poly(fluorinated ether) diols, short chain polyester diols, short chain polyacrylate diols, short chain polymethacrylate diols, and short chain poly(vinyl aromatic) diols.

In certain embodiments, the biostability and/or biocompatibility of the polyisobutylene urethane copolymers in accordance with the invention may be improved by end-capping the copolymers with short aliphatic chains (e.g., $[-CH_2]_n-CH_3$ groups, $[-CH_2]_n-C(CH_3)_3$ groups, $[-CH_2]_n-CF_3$ groups, $[-CH_2]_n-C(CF_3)_3$ groups, $[-CH_2]_n-CH_2OH$ groups, $[-CH_2]_n-C(OH)_3$ groups and $[-CH_2]_n-C_6H_5$ groups, etc., where n may range, for example, from 1 to 2 to 5 to 10 to 15 to 20, among others values) that can migrate to the polymer surface and self assemble irrespective of synthetic process to elicit desirable immunogenic response when implanted in vivo. Alternatively, a block copolymer or block terpolymer with short aliphatic chains (e.g., $[-CH_2]_n$-*b*-$[-CH_2O]_n-CH_3$ groups, $[-CH_2]_n$-*b*-$[-CH_2O]_n-CH_2CH_2C(CH_3)_3$ groups, $[-CH_2]_n$-*b*-$[-CH_2O]_n-CH_2CH_2CF_3$ groups, $[-CH_2]_n$-*b*-$[-CH_2O]_n-CH_2CH_2C(CF_3)_3$ groups, $[-CH_2]_n$-*b*-$[-CH_2O]_n-CH_2CH_2OH$ groups, $[-CH_2]_n$-*b*-$[-CH_2O]_n-C(OH)_3$ groups, $[-CH_2]_n$-*b*-$[-CH_2O]_n-CH_2CH_2-C_6H_5$ groups, etc., where n may range, for example, from 1 to 2 to 5 to 10 to 15 to 20, among others values) that can migrate to the surface and self assemble can be blended with the copolymer toward the end of synthesis. These end-capping segments may also help to improve the thermal processing of the polymer by acting as processing aids or lubricants. Processing aids, antioxidants, waxes and the like may also be separately added to aid in thermal processing.

Various techniques may be employed to synthesize the polyisobutylene urethane copolymers from the diol and diisocyanate starting materials. The reaction may be conducted, for example, in organic solvents or using supercritical $CO_2$ as a solvent. Ionomers can be used for polymer precipitation.

In certain other embodiments, a one step method may be employed in which a first macrodiol (M1) (e.g., a polymeric diol such as an unsaturated or a saturated polyisobutylene diol,), a second macrodiol (M2) (e.g., a polyether diol) and a diisocyante (DI) (e.g., MDI, TDI, etc.) are reacted in a single step. Molar ratio of diisocyanate relative to the first and second diols is 1:1. For example, the ratio DI:M1:M2 may equal 2:1:1, may equal 2:1.5:0.5, may equal 2:0.5:1.5, among many other possibilities. Where a ratio of DI:M1:M2 equal to 2:1:1 is employed, a polyurethane having the following structure may be formed $-[DI-M1-DI-M2-]_n$ although the chains are unlikely to be perfectly alternating as shown. In some embodiments, a chain extender is added to the reaction mixture, such that the molar ratio of diisocyanate relative to the first and second macrodiols and chain extender is 1:1. For example, the ratio DI:M1:M2:CE may equal 4:1:1:2, may equal 2:0.67:0.33:1, may equal 2:0.33:0.67:1, or may equal 5:1:1:3, among many other possibilities. Where a ratio of DI:M1:M2:CE equal to 4:1:1:2 is employed, a polyurethane having the following structure may be formed $-[DI-M1-DI-CE-DI-M2-DI-CE-]_n$, although the chains are unlikely to be perfectly alternating as shown.

In some embodiments, a two-step method is employed in which first and second macrodiols and diisocyante are reacted in a ratio of DI:M1:M2 of ≥ 2:1:1 in a first step to form isocyanate capped first and second macrodiols, for example DI-M1-DI and DI-M2-DI. In a second step, a chain extender is added which reacts with the isocyanate end caps of the macrodiols. In some embodiments, the number of moles of hydroxyl or amine groups of the chain extender may exceed the number of moles of isocyanate end caps for the macrodiols, in which case additional diisocyante may be added in the second step as needed to maintain a suitable overall stoichiometry. As above, the molar ratio of diisocyanate relative to the total of the first macrodiol, second macrodiol, and chain extender is typically 1:1, for example, DI:M1:M2:CE may equal 4:1:1:2, which may in theory yield an idealized polyurethane having the following repeat structure $-[DIM1-DI-CE-DI-M2-DI-CE-]_n$, although the chains are unlikely to be perfectly alternating as shown. In other examples, the DI:M1:M2:CE ratio may equal 4:1.5:0.5:2 or may equal 5:1:1:3, among many other possibilities.

In some embodiments, three, four or more steps may be employed in which a first macrodiol and diisocyante are reacted in a first step to form isocyanate capped first macrodiol, typically in a DI:M1 ratio of ≥2:1 such that isocyanate end caps are formed at each end of the first macrodiol (although other ratios are possible including a DI:M1 ratio of 1:1, which would yield an average of one isocyanate end caps per macrodiol). This step is followed by second step in which the second macrodiol is added such that it reacts with one or both isocyanate end caps of the isocyanate capped first macrodiol. Depending on the relative ratios of DI, M1 and M2, this step may be used to create structures (among other statistical possibilities) such as M2-DIM1-DI-M2 (for a DI:M1:M2 ratio of 2:1:2), M2-DI-M1-DI (for a DI:M1:M2 ratio of 2:1:1), or M1-DI-M2 (for a DI:M1:M2 ratio of 1:1:1).

In certain embodiments, a mixed macrodiol prepolymer, such as one of those in the prior paragraph, among others (e.g., M2-DI-M1-DI-M2, M1-DI-M2-DI-M1, DI-M1-DI-M2, etc.) is reacted simultaneously with a diol or diamine chain extender and a diisocyanate, as needed to maintain stoichiometry. For example, the chain extension process may be used to create idealized structures along the following lines, among others: $-[DI-M2-DI-M1-DI-M2-DI-CE-]_n$, -[DI-M1-

DI-M2-DI-M1-DI-CE-]$_n$ or -[DI-M1-DIM2-DI-CE-]$_n$, although it is again noted that the chains are not likely to be perfectly alternating as shown.

In certain other embodiments, a mixed macrodiol prepolymer is reacted with sufficient diisocyanate to form isocyanate end caps for the mixed macrodiol prepolymer (e.g., yielding DI-M2-DI-M1-DI-M2-DI, DI-M1-DI-M2-DI-M1-DI or DI-M1-DIM2-DI, among other possibilities). This isocyanate-end-capped mixed macrodiol can then be reacted with a diol or diamine chain extender (and a diisocyanate, as needed to maintain stoichiometry). For example, the isocyanate-end-capped mixed macrodiol can be reacted with an equimolar amount of a chain extender to yield idealized structures of the following formulae, among others: -[DI-M2-DI-M1-DI-M2-DI-CE-]$_n$, -[DI-M1-DI-M2-DI-M1-DI-CE-]$_n$ or -[DI-M1-DI-M2-DI-CE-]$_n$.

As noted above, chain extenders can be employed to increase the ratio of hard segment material to soft segment material in the urethane, urea or urethane/urea polymers described herein, which can in turn result in a polymer with higher modulus, lower elongation at break and increased strength. For instance the molar ratio of soft segment to chain extender to diisocyanate (SS:CE:DI) can range, for example, from 1:9:10 to 2:8:10 to 3:7:10 to 4:6:10 to 5:5:10 to 6:4:10 to 7:3:10 to 8:2:10 to 9:1:10 to 10:0:10, among other values.

In a particular embodiment, the soft segment of the polyisobutylene urethane copolymer is formed from a first soft macrodiol or macrodiamine (M1) and second soft macrodiol or macrodiamine (M2) in a molar ratio of M1 to M2 (M1:M2) from 99:1 to 95:5 to 90:10 to 75:25 to 66:33 to 50:50 to 25:75 to 10:90 to 5:95 to 1:99, more particularly, from 90:10 to 85:15 to 80:20 to 75:25 to 70:30 and most particularly from about 75:25 to about 50:50.

Exemplary number average molecular weights for M1 and M2 may range from 100 to 10000, more preferably 200 to 5000, most preferably 750 to 2500. Exemplary materials for M1 include polyisobutylene diols, whereas preferred materials for M2 include polyether diols such as polytetramethylene oxide (PTMO) diol and polyhexamethylene oxide (PHMO) diol. In certain embodiments, M1 is polyisobutylene diol having a number average molecular weight between about 1000 and 5000 and M2 is PTMO having a number average molecular weight of about 900 and 1200.

The molar ratio and number average molecular weight of the diol starting materials may be used to calculate the weight ratio of first to second soft segments in the polyisobutylene urethane copolymer. For example, if 48.00 g polyisobutylene diol having a number average molecular weight of 1000 is reacted with 32.00 g PTMO having a number average molecular weight of 1000, the weight ratio polyisobutylene segment to PTMO segment would be 60:40. In embodiments in which the soft segments include polyisobutylene and polytetramethylene oxide, the resulting weight ratio ranges from 15:1 to 13:1 to 12:1 to 7.5:1 to 4.5:1 to 3:1 to 2:1 to 3:2 to 1:1 to 1:2 to 2:3, more particularly, from about 99:1 to 95:5 to 90:10 to 80:20 to 70:30.

In another embodiment, the ratio of PIB diol to polytetramethylene oxide diol included in the reaction mixture results in a polyisobutylene urethane copolymer having soft segments comprising no more than about 30 wt% polytetramethylene oxide, particularly between about 10 wt% and 30 wt% polytetramethylene oxide, more particularly between about 5 wt% and about 20 wt% polytetramethylene oxide and even more particularly between about 10 wt% and about 20 wt% polytetramethylene oxide based on the total weight of soft segment. The balance of the soft segment weight may comprise polyisobutylene. In other embodiments, the soft semgnet may not comprise polytetramethylene oxide. For example, the soft segment may comprise 100 wt% polyisobutylene.

In a further embodiment, the ratio of PIB diol to polyhexamethylene oxide diol included in the reaction mixture results in a polyisobutylene urethane copolymer having soft segments comprising no more than about 30 wt% polyhexamethylene oxide, particularly between about 10 wt% and 30 wt% polyhexamethylene oxide, more particularly between about 15 wt% and 25 wt% polyhexamethylene oxide and even more particularly between about 20 wt% and 25 wt% polyhexamethylene oxide based on the total weight of soft segment. The balance of the soft segment weight may comprise polyisobutylene.

Polyisobutylene urethane copolymers containing PTMO of no more than about 30% show minimal decrease in both weight and tensile strength and exhibit a continuous surface morphology when subjected to accelerated degradation testing indicating favorable biostability of these materials. Additionally, when the amount of polyether diol (e.g. PTMO) is increased, the degradation also increases, suggesting that a low PTMO content promotes biostability.

In yet another exemplary embodiment, the ratio of PIB diol to polyether diol (e.g., polytetramethylene oxide diol or polyhexamethylene diol) to polydimethylsiloxane diol included in the reaction mixture results in a polyisobutylene urethane, urea or urethane/urea copolymer having a weight ratio of polyisobutylene to polyether to polydimethylsiloxane ranging from about 60:20:20 to about 80:15:5.

The fibrous matrix 40 may be formed using several different techniques, such as electrospinning and melt blowing. In some embodiments, smaller fiber sizes may be achieved using electrospinning. Figures 3 and 4 schematically illustrate both techniques.

Figure 3 provides a schematic illustration of electrospinning. An electric field may be used to draw a polymer solution or melt 54 from a capillary source 52. In some embodiments, the capillary source 52 may be a syringe. The polymer solution or melt 54 is drawn to a grounded collector 58. A high voltage power supply 56 may be used to power the process. The elements 60 to be coated may be placed on the collector 58 to be coated. Upon drying, a thin polymeric

web 62 may be formed. In some embodiments, the fiber sizes may be controlled by adjusting the relative concentration of polymer in the polymer solution or melt 54.

The elements 60 may be rotated and the linear speed of the surface can be calculated based on the diameter of element 60 and the rotational speed. As element 60 rotates, the capillary source 52 may move longitudinally over element 60. A cycle is equal to two passes of the capillary source 52 over element 60.

The concentration of polymer in the electrospinning solution and solvent selection are important factors in achieving desired fibrous matrix properties, and in particular for controlling porosity and/or fiber size. Additionally, a small amount of a metal salt solution may be added to the electrospinning solution to improve deposition. In certain embodiments, the electrospinning solution has a polymer concentration of between about 1 wt% and about 40 wt%, more particularly between about 1 wt% and about 30 wt%, even more particularly from about 2 wt% to about 25 wt%. In another example, salt is added to achieve an electrospinning solution having a conductivity of at least about 100 μS/cm, and more preferably, about 100 μS/cm. Suitable solvents include dimethylformamide, dimethylacetamide, *N*-methyl-2-pyrrolidone, dimethyl sulfoxide, acetone, cyclohexane, tetrohydrofuran, pyridine, chloroform as well as mixtures and co-solvents thereof.

Figure 4 provides a schematic illustration of meltblowing. An apparatus 70 is configured to accommodate a polymer melt 72. The polymer melt 72 passes through an orifice 74 and is carried through the orifice 74 via streams of hot air 76 that pass through the apparatus 70. As the polymer melt 72 exits the orifice 74, they are met with streams of heated air 78 that helps elongate the polymer melt 72. As a result, the polymer melt 72 forms fibers 80 that impinge onto a collector 82. An element to be coated may simply be placed on or in front of the collector 82.

In certain embodiments, the lead 10 may be assembled before the fibrous matrix 40 is formed directly on the lead 10. In other embodiments, the fibrous matrix 40 may be formed on a component of the lead 10 before the lead 10 is assembled. In still further embodiments, the fibrous matrix 40 may be separately formed and then subsequently disposed onto a portion of the lead 10.

In certain embodiments, the fibrous matrix may be formed from more than one polymer material in the form of a composite or material layers. For example in certain embodiments, a first layer comprising a first polymer material may be deposited onto a portion of the lead 10, followed by a second layer formed by a second polymer material. Additional layers may also be applied as desired. In another example, one of a plurality of layers comprises a non-conductive polymer material while another of the plurality of layers comprises a conductive material. In a further example, each layer comprises a non-conductive material.

In embodiments where one or more therapeutic agents are provided, they may be positioned beneath, blended with, or attached to (e.g., covalently or non-covalently bound to) polymeric regions (e.g., lead components) in accordance with the invention. "Therapeutic agents," "drugs," "pharmaceutically active agents," "pharmaceutically active materials," and other related terms may be used interchangeably herein.

A variety of therapeutic agents can be employed in conjunction with the present invention including the following among others: (a) anti-inflammatory agents such as dexamethasone, prednisolone, corticosterone, budesonide, estrogen, sulfasalazine and mesalamine, (b) antimicrobial agents such as triclosan, cephalosporins, aminoglycosides and nitrofurantoin; (c) anesthetic agents such as lidocaine, bupivacaine and ropivacaine, (d) anti-proliferative agents such as paclitaxel, (e) immunosuppressants such as sirolimus, biolimus and everolimus, (f) anti-thromobogenic agents such as heparin, and (g) growth factors such as VEGF.

Where a therapeutic agent is present, a wide range of loadings may be used in conjunction with the medical devices of the present invention. Typical therapeutic agent loadings range, for example, from than 1 wt% or less to 2 wt% to 5 wt% to 10 wt% to 25 wt% or more of the fibrous matrix.

Moreover, in some embodiments, a part of the lead body or the complete lead body can be further coated with a lubricious coating, typically formed from a hydrophilic polymer or other material (e.g., poly(vinyl pyrrolidone), polyethylene/oligoethylene, polyHEMA, polytetraglyme, hyalorunic acid and its derivatives, chitosan and its derivatives, etc.), which material may be crosslinked, to reduce coefficient of friction.

Experimental Section

Two polyisobutylene polyurethane, PIB-PUR-013 and PIB-PUR-016, were prepared and used in the following Examples. The polyisobutylene polyurethanes were prepared by synthesizing polyisobutylene diol which was used in the synthesis of the polyisobutylene polyurethanes.

Synthesis of polyisobutylene (PIB) diol

Materials Used

Titanium tetrachloride (Aldrich #208566), 2,6-di-tert-butylpyridine (DTBP, Aldrich, 99.4 %), allyltrimethylsilane

(ATMS, Gelest#SIA0555.0) and hindered dicumyl ether (HDCE, initiator) were all prepared in separate bottles in anhydrous hexanes

(Aldrich #227064) solution under nitrogen environment to avoid moisture. 9-BBN (Aldrich, 0.5 M in tetrahydrofuran), sodium hydroxide (NaOH, VWR, 3N solution), isobutylene (IB, Conley Fas Ltd, 99.5 %), methyl chloride (MeCI, Metheson, 99.9 %), methanol (MeOH, VWR, BDH1135), anhydrous tetrahydrofuran (THF, Aldrich #401757), potassium carbonate ($K_2CO_3$, Aldrich #209619) and hydrogen peroxide ($H_2O_2$, VWR, 30 wt% solution in water, ACS reagent) was used as received.

Polymerizations were carried out in 30 gallon Hastelloy reactor under constant nitrogen purge. Required amounts of hexanes (31 kilograms (kg)), HDCE (743 grams (gm)) and 2,6-di-*tert*-butylpyridine (100 gm, DTBP) were added to the reactor. MeCl (29.5 kg) and isobutylene (4.5 kg, IB) were added to the reactor through a cooling coil that was cooled to -80 °C. The solution was stirred thoroughly by overhead agitator and kept at -80 °C by liquid nitrogen cooling jacket in the reactor. The polymerization of IB was initiated by adding $TiCl_4$ (1519 gm). After 30 minutes, the capping reaction of $PIB^+$ cation with ATMS (1220 gm) was carried out at -80 °C to introduce allyl group in the end of polymer chains. After 2 hours, the reactions were terminated by the addition of MeOH at -80 °C. The resulting solution was stirred at 25 °C overnight until MeCl was evaporated completely. The polymer was washed with Sodium Chloride water solution. Waste from bottom was decanted and followed by DI water wash. Multiple water washes were done until the pH of waste reached neutral. After the final wash, it was precipitated in large amount of MeOH and MeOH was decanted. The resulting polymer was transferred to distillation flask and mixed with toluene and dried by azeotropic distillation of dry toluene (10 liters (L)). After removing toluene, anhydrous THF (10L) was added. The resulting product was PIB diallyl.

The PIB diallyl (4918 g, 0.00204 mol, Mn = 2,560 grams per mole (g/mol)) was charged to 100 gallon stainless steel reactor. Extra anhydrous THF (54 kg) was added to the tank and mixed. After complete dissolution, 9-BBN (21.53 kg, 0.5mol in THF) was added and agitated for 5 hours under continuous nitrogen purge. Deionized water (5.74 kg) was added using a pump to the reaction mixture. Next charge was 3mol NaOH (1.1 kg) solution. Temperature was monitored throughout the reaction process and always kept under 35 °C by using cooling water jacket in the reactor tank. A 30 wt% $H_2O_2$ (1.08 kg) mixture was then pumped in slowly. The reaction mixture turned milky. The reaction was allowed to continue for 12 hours under nitrogen purge and agitation. After the reaction is complete excess amount of hexanes was added to the reaction mixture. The bottom aqueous layer was removed by draining from the bottom. $K_2CO_3$ (aqueous) was added to the remaining hexane layer and extracted out. The hexane layer was then washed with distilled water 3 times. The hexane was then evaporated off to form a concentrated PIB solution. To this MeOH was added and the polyisobutylene (PIB) was allowed to settle at the bottom. The top MeOH layer was decanted and the PIB was re-dissolved in hexane to form a concentrated solution. Again, MeOH was added and PIB was collected at the bottom. The resulting polymer was transferred to distillation flask and mixed with toluene and dried by azeotropic distillation of dry toluene (10 L). The resulting product was PIB diol (PIB, ~2000 g/mol).

Synthesis of polyisobutylene polyurethane (PIB-PUR)

Materials Used

4,4'-methylene-bis(phenyl isocyanate) (MDI, crystalline, Aldrich, 98 %), poly(tetramethylene oxide)diol (PTMO, Aldrich, 1000 g/mol) were used as received. Polyisobutylene diol (PIB, ~2000 g/mol) as above. The diols were dried under vacuum for 24-72 hours at 50-100 °C. 1,4-butanediol (BDO, Alfa-Aesar, 99 %) was dried by refluxing it overnight over $CaH_2$ followed by distillation under vacuum at 190 °C. The reaction was catalyzed by Tin-octoate ($Sn(Oct)_2$, Aldrich, 99 %).

Toluene (Aldrich 99.5 %) and tetrahydrofurane (THF, Aldrich, 99 %) were dried by refluxing them over benzophenone (Sigma-Aldrich, 99 %) and Na metal (Sigma-Aldrich, 99.9 %) overnight and distilled under nitrogen atmosphere. For dissolving the polyurethane samples 2 wt/wt% tetra-N-butylammonium bromide (TBAB, Alfa Aesar, 98 %) in distilled THF solution was used as solvent.

Polyurethane (PIB-PTMO-PU) was synthesized from MDI, PIB, PTMO, BDO and $Sn(Oct)_2$. The basic composition of the materials is shown in Table 1. The amounts of the components were calculated based on the composition markers with the equations Eq. 1 - 4. The NCO/OH showed the ratio of the isocyanate and hydroxyl groups, the SS/HS was the amount of the soft segments (polyols) vs. the hard segments (MDI and BDO), and PIB/PTMO ratio was the ratio of the two different polyols in weight percentage to weight percentage (wt%/wt%).

**Table 1** Basic composition of the PIB-PTMO-PU materials synthesized.

| Composition marker | | Value |
|---|---|---|
| NCO/OH | (n/n) | 1.05 |

(continued)

| Composition marker | | Value |
|---|---|---|
| SS/HS | (wt%/wt%) | 65/35 |
| PIB/PTMO | (wt%/wt%) | 80/20 |

(1) $$m(PU) = m(MDI) + m(PIB) + m(PTMO) + m(BDO)$$

(2) $$NCO/OH = \frac{n(MDI)}{n(PIB) + n(PTMO) + n(BDO)}$$

(3) $$SS/HS = \frac{m(PIB) + m(PTMO)}{m(MDI) + m(BDO)}$$

(4) $$PIB/PTMO = \frac{m(PIB)}{m(PTMO)}$$

Synthesis of the materials was carried out with a pre-polymer synthesis method. The polyols were measured precisely into a 100 ml three neck round bottom flask, equipped with a distilling receiver and a condenser. To the polyols, 50 ml toluene was added and refluxed overnight at 110 °C for drying. The next day the toluene was evaporated completely by nitrogen purging, and the dry polyols were cooled down to room temperature.

After the drying the polyols the distilling receiver was changed to a mechanical stirrer (200 revolutions per minute (rpm)), the diols were dissolved in 10 ml distilled toluene again, and the calculated amount of crystalline MDI was added to the mixture. PIB-PUR-013 was produced without the addition of process aides in the mixture; PIBPUR-016 was produced with the addition of process aides in the mixture. After 5 min stirring at 25 °C the mixture was heated to 100 °C and the pre-polymer synthesis was continued for 2 hours under dry nitrogen atmosphere. Chain extender (BDO) was added to the pre-polymer solution followed immediately by the Sn-octoate catalyst. The reaction was continued for the 2 more hours. At the end of the chain extension step we switched off the stirring, and cooled down the material in an hour to 50 °C. The chilled polymer was removed from the flask to a mold, cured at ambient conditions for 7 days followed by a vacuum drying overnight at 70 °C.

Evaluation of Properties

Compression molding

For the tensile testing of the materials, polymers were compression molded between Teflon coated aluminum foils on a Carver model 'C' press at 170 °C for 10 min (3 min preheating and 7 min pressing) using 80 kilonewtons (kN) load. The dimensions of the mold were 70 x 60 x 0.3 mm. During the preheating time the pressure was increased carefully and 5 aeration was occurred at 20, 40, 60, 80 and again 80 kN load. After 10 min the load was released, the sample was removed and quenched to room temperature.

Gel permeation chromatography

Molecular weights and structures of the polyurethane samples were characterized with gel permeation chromatography (GPC) using a Waters HPLC system equipped with a model 515 HPLC pump, model 2414 differential refractometer, model 486 absorbance detector, on-line multiangle laser light scattering (MALLS) detector (MiniDawn Treos, Wyatt Technology Inc.), Model 717 sample processor, and five Ultrastyragel GPC columns connected in the following series: 500, $10^3$, $10^4$, $10^5$, and 100A. The carrier solvent used was 2 wt/wt% TBAB:THF with the flow rate of 1 mL/min. The samples were prepared by the dissolving of 0.020 g polymer in 2 ml-s of the TBAB:THF solution used as eluent for GPC system. The samples were filtered on 0.45 $\mu$m filters before the measurement. The results for PIB-PUR-013 and PIB-PUR-016 are shown in Table 2.

Table 2

| Sample | Mn | Mw | PDI |
|---|---|---|---|
| | g/mol | g/mol | |
| PIB-PUR-013 | 177000 | 336000 | 1.90 |
| PIB-PUR-016 | 118000 | 268000 | 2.25 |

Differential scanning calorimetry

The thermal properties of the polymers were analyzed by differential scanning calorimetry (DSC) with a Q2000 from TA instruments, using T-zero pans. Temperature was ramped from -80 °C to 280 °C, at a ramp rate of 5 °C per minute. Graphical illustration of the DSC results for PIB-PUR-013 are presented in Figure 5 and graphical illustration of the DSC results for PIB-PUR-016 are presented in Figure 6.

Tensile testing

Mechanical properties of the samples were analyzed by tensile testing of dog-bone type specimens with 25 x 3.4 x 0.4 mm dimensions (ASTM D412 standard) at 50 mm/min cross head speed using an Instron 4400R apparatus. Ultimate tensile strength and break strain values were derived from the force vs. elongation curves. All tests were carried out according to ASTM D412 standard at ambient (25 °C, 50 percent relative humidity) conditions. The results for PIB-PUR-013 and PIB-PUR-016 are shown in Table 3.

Table 3

| Sample | Ultimate tensile strength (MPa) | Strain at break (%) |
|---|---|---|
| PIB-PUR-013 | 20 | 318 |
| PIB-PUR-016 | n/a | n/a |

Example 1

PIB-PUR-013 (80A grade; prepared as described above) was dissolved in pyridine to form an 8 wt% PIB-PUR solution. The PIB-PUR solution was electrospun using an electrospinning apparatus onto a shock coil component. A 22-gauge (0.04 inch diameter) needle was the capillary source. The shock coil component was rotating at a linear surface speed of about 69 inches per minute (in/min). The voltage, solution flow rate and distance from the needle to the collector were varied during the process to maintain the polymer melt. The voltage was varied between about 6 and 9 kilovolts (kV) (nominally about 7.5 kV), the solution flow rate was nominally about 2.2 milliliters per hour (mL/hr), and the distance from needle to the collector was nominally about 15 centimeters (cm). A total of 150 cycles were completed at 22 °C and 36 percent relative humidity. The average diameter of the fibers formed was 1.07 microns. The process parameters used are presented in Table 4. An image of the fibrous matrix is shown in Figure 7.

Table 4

| Process Parameter | Value |
|---|---|
| Voltage | 6 to 9 kV, nominally 7.5 kV |
| Linear surface speed | nominally 69 in/min |
| Solution flow rate | nominally 2.2 mL/hr |
| Distance from Needle tip to collector | 15 cm |
| Number of cycles | 150 |
| Temperature | 22 °C |
| Relative Humidity | 36 percent |

Example 2

PIB-PUR-016 (80A grade) was dissolved in pyridine to form a 8 wt% PIB-PUR solution. The PIB-PUR solution was electrospun using an electrospinning apparatus onto a shock coil component. A 22-gauge (0.04 inch diameter) needle was the capillary source. The shock coil component was rotating at a linear surface speed of about 69 in/min. The voltage, solution flow rate and distance from the needle to the collector were varied during the process to maintain the polymer melt. The voltage was nominally about 8.0 kV, the solution flow rate was nominally about 0.6 mL/hr, and the distance from needle to the collector was nominally about 12.5 cm. A total of 300 cycles were completed at 23 °C and 24 percent relative humidity. The average diameter of the fibers formed was 0.57 microns. The process parameters used are presented in Table 5.

Table 5

| Process Parameter | Value |
|---|---|
| Voltage | Nominally 8.0 kV |
| Linear surface speed | nominally 69 in/min |
| Solution flow rate | nominally 0.6 mL/hr |
| Distance from Needle tip to collector | 12.5 cm |
| Number of cycles | 300 |
| Temperature | 23 °C |
| Relative Humidity | 24 percent |

Example 3

PIB-PUR-016 (80A grade) was dissolved in pyridine to form a 15 weight percent PIB-PUR solution. The PIB-PUR solution was electrospun using an electrospinning apparatus onto two shock coils contained on a lead. The apparatus included a 22-gauge (0.04 inch diameter) needle as the capillary source. The process parameters used are presented in Table 6. The average diameter of the fibers formed was 1.74 microns.

Table 6

| Process Parameter | Value (coil 1) | Value (coil 2) |
|---|---|---|
| Voltage | 8.57 kV | 8.75 kV |
| Linear surface speed | 69 in/min | 69 in/min |
| Solution flow rate | 0.6 mL/hr | 0.6 mL/hr |
| Distance from Needle tip to collector | 20 cm | 20 cm |
| Number of cycles | 300 | 300 |
| Temperature | 23 °C | 23 °C |
| Relative Humidity | 24 percent | 24 percent |

Example 4

PIB-PUR-016 (80A grade) was dissolved in pyridine to form a 15 wt% solution. The PIB-PUR solution was electrospun using an electrospinning apparatus onto two shock coils contained on a lead with a 22 gauge (0.04 inch diameter) needle as the capillary source. The process parameters used are presented in Table 7. The average diameter of the fibers formed was 1.78 microns.

Table 7

| Process Parameter | Value (coil 1) | Value (coil 2) |
|---|---|---|
| Voltage | 8.57 kV | 8.75 kV |

(continued)

| Process Parameter | Value (coil 1) | Value (coil 2) |
|---|---|---|
| Linear surface speed | 69 in/min | 69 in/min |
| Solution flow rate | 0.6 mL/hr | 0.6 mL/hr |
| Distance from Needle tip to collector | 20 cm | 20 cm |
| Number of cycles | 300 | 300 |
| Temperature | 23 °C | 23 °C |
| Relative Humidity | 24 percent | 24 percent |

Example 5

PIB-PUR-016 (80A grade) was dissolved in pyridine to form a 15 wt% solution. The PIB-PUR solution was electrospun using an electrospinning apparatus onto two shock coils contained on a lead with a 22 gauge (0.04 inch diameter) needle as the capillary source. The process parameters used are presented in Table 8. The average diameter of the fibers formed was 2.22 microns.

Table 8

| Process Parameter | Value (coil 1) | Value (coil 2) |
|---|---|---|
| Voltage | 8.57 kV | 8.75 kV |
| Linear surface speed | 69 in/min | 69 in/min |
| Solution flow rate | 0.6 mL/hr | 0.6 mL/hr |
| Distance from Needle tip to collector | 20 cm | 20 cm |
| Number of cycles | 300 | 300 |
| Temperature | 23°C | 23°C |
| Relative Humidity | 24 % | 24 % |

**Example 6**

PIB-PUR-23, which included 65 % by weight soft segments and 35% by weight hard segments, was dissolved in pyridine to form a 8 wt% solution. The PIB-PUR solution was electrospun using an electrospinning apparatus onto a substrate.

The PIB-PUR/pyridine was dispensed at 0.5 mL/hr per hour. The nozzle to target distance was held at 12.5 cm. An image of the fibrous matrix formed in Example 6 is shown in Figure 8. The average diameter of the fibers was 650 nanometers.

Various modifications and additions can be made to the exemplary embodiments discussed without departing from the scope of the present invention. For example, while the embodiments described herein refer to particular features, the scope of this invention also includes embodiments having different combinations of features and embodiments that do not include all of the described features. Accordingly, the scope of the present invention is intended to embrace all such alternatives, modifications, and variations as fall within the scope of the claims.

## Claims

**1.** A medical electrical lead comprising:

an insulative lead body extending from a distal region to a proximal region;
a conductor disposed within the insulative lead body and extending from the proximal region to the distal region;
an electrode disposed on the insulative lead body and in electrical contact with the conductor; and
a non-conductive fibrous matrix disposed at least partially over the electrode,
the fibrous matrix comprising a polyisobutylene urethane, a polyisobutylene urea or a polyisobutylene urethane/urea copolymer comprising 10% to 60% by weight of a hard segment and the balance by weight of a soft segment, and wherein the fibrous matrix comprises a plurality of randomly aligned fibers having an average fiber diameter ranging from 10 nanometers to 400 nanometers.

**2.** The medical electrical lead of claim 1, wherein the hard segment includes a polyisobutylene urethane, polyisobutylene urea or polyisobutylene urethane/urea, and wherein the soft segment includes at least one polyisobutylene macrodiol or diamine.

**3.** The medical electrical lead of claim 1, wherein the fibrous matrix has a porosity of at least 40%, an average pore size ranging from 10 nanometers to 10 microns and a thickness ranging from 1 nanometer to 1000 microns.

**4.** The medical electrical lead of claim 1, wherein the fibrous matrix has a porosity of at least 40%, 60%, 75% or 80%.

**5.** A method of forming a medical electrical lead having an insulative lead body and an electrode disposed on the insulative lead body, the method comprising steps of:

forming a fibrous matrix comprising at least one polyisobutylene urethane, a polyisobutylene urea or a polyisobutylene urethane/urea copolymer by electrospinning or meltblowing; and
disposing the fibrous matrix at least partially over the electrode;
wherein the fibrous matrix comprising a polyisobutylene urethane, a polyisobutylene urea or a polyisobutylene urethane/urea copolymer comprising 10% to 60% by weight of a hard segment and a balance by weight of a soft segment, and wherein the fibrous matrix comprises a plurality of randomly aligned fibers having an average fiber diameter ranging from 10 nanometers to 400 nanometers.

**6.** The method of claim 5, wherein forming the fibrous matrix comprises electrospinning the polyisobutylene urethane, polyisobutylene urea or polyisobutylene urethane/urea copolymer.

**7.** The method of claim 5, wherein prior to electrospinning a coating solution comprising between 1 and 30 wt% polyisobutylene urethane, polyisobutylene urea or polyisobutylene urethane/urea copolymer is prepared.

**8.** The method of claim 5, wherein prior to electrospinning a coating solution comprising between 2 and 25 wt% polyisobutylene urethane, polyisobutylene urea or polyisobutylene urethane/urea copolymer is prepared.

**9.** The method of claim 5, wherein forming the fibrous matrix comprises melt blowing the polyisobutylene urethane, polyisobutylene urea or polyisobutylene urethane/urea copolymer.

**10.** The method of claim 5, wherein forming the fibrous matrix comprises forming the fibrous matrix on a substrate and depositing the fibrous matrix at least partially over the electrode.

**11.** The method of claim 5, wherein forming the fibrous matrix comprises forming the fibrous matrix at least partially over the electrode.

**12.** The medical electrical lead of claim 1, wherein the fibrous matrix has an average fiber size ranging from 10 nanometers to 400 nanometers, a porosity of at least 40%, an average pore size ranging from 70 nanometers to 250 nanometers and a thickness ranging from 10 nanometers to 10 microns.

**13.** The medical electrical lead of claim 1, wherein the fibrous matrix has an average fiber size ranging from 400 nanometers to 1000 nanometers, a porosity of at least 40%, an average pore size ranging from 1 micron to 100 microns and a thickness ranging from 10 nanometers to 10 microns.

**14.** The medical electrical lead of claim 1, wherein the soft segment includes at least 70% by weight of at least one polyisobutylene macrodiol and/or diamine.

## Patentansprüche

**1.** Medizinische elektrische Leitung, umfassend:

einen isolierenden Leitungskörper, der sich von einer distalen Region zu einer proximalen Region erstreckt;
einen Leiter, der innerhalb des isolierenden Leitungskörpers angeordnet ist und sich von der proximalen Region zur distalen Region erstreckt;
eine Elektrode, die an dem isolierenden Leitungskörper angeordnet ist und mit dem Leiter in elektrischem Kontakt steht; und
eine nicht-leitende Fasermatrix, die zumindest zum Teil über der Elektrode angeordnet ist,
wobei die Fasermatrix ein Polyisobutylen-Urethan, einen Polyisobutylen-Harnstoff oder ein Polyisobutylen-Urethan/Harnstoff-Copolymer umfasst, das bzw. der zu 10 Gewichtsprozent bis 60 Gewichtsprozent ein hartes Segment und zu übrigen Gewichtsteilen ein weiches Segment umfasst, und wobei die Fasermatrix eine Vielzahl von zufällig ausgerichteten Fasern umfasst, die einen durchschnittlichen Faserdurchmesser im Bereich von 10 Nanometern bis 400 Nanometern aufweisen.

**2.** Medizinische elektrische Leitung nach Anspruch 1, wobei das harte Segment ein Polyisobutylen-Urethan, einen Polyisobutylen-Harnstoff oder Polyisobutylen-Urethan/Harnstoff enthält, und wobei das weiche Segment mindestens ein Polyisobutylen-Makrodiol oder -Diamin enthält.

**3.** Medizinische elektrische Leitung nach Anspruch 1, wobei die Fasermatrix eine Porosität von mindestens 40 %, eine durchschnittliche Porengröße im Bereich von 10 Nanometern bis 10 Mikrometern und eine Dicke im Bereich von 1 Nanometer bis 1000 Mikrometern aufweist.

**4.** Medizinische elektrische Leitung nach Anspruch 1, wobei die Fasermatrix eine Porosität von mindestens 40 %, 60 %, 75 % oder 80 % aufweist.

**5.** Verfahren zum Ausbilden einer medizinischen elektrischen Leitung, die einen isolierenden Leitungskörper und eine Elektrode aufweist, die an dem isolierenden Leitungskörper angeordnet ist, wobei das Verfahren als Schritte umfasst:

Ausbilden einer Fasermatrix, die mindestens ein Polyisobutylen-Urethan, einen Polyisobutylen-Harnstoff oder ein Polyisobutylen-Urethan/Harnstoff-Copolymer umfasst, durch Elektrospinnen oder Schmelzblasen; und
Anordnen der Fasermatrix zumindest zum Teil über der Elektrode;
wobei die Fasermatrix ein Polyisobutylen-Urethan, einen Polyisobutylen-Harnstoff oder ein Polyisobutylen-Urethan/Harnstoff-Copolymer umfasst, das bzw. der zu 10 Gewichtsprozent bis 60 Gewichtsprozent ein hartes Segment und zu übrigen Gewichtsteilen ein weiches Segment umfasst, und wobei die Fasermatrix eine Vielzahl von zufällig ausgerichteten Fasern umfasst, die einen durchschnittlichen Faserdurchmesser im Bereich von 10 Nanometern bis 400 Nanometern aufweisen.

**6.** Verfahren nach Anspruch 5, wobei das Ausbilden der Fasermatrix ein Elektrospinnen des Polyisobutylen-Urethans, des Polyisobutylen-Harnstoffs oder des Polyisobutylen-Urethan/Harnstoff-Copolymers umfasst.

**7.** Verfahren nach Anspruch 5, wobei vor dem Elektrospinnen eine Beschichtungslösung hergestellt wird, die zwischen 1 und 30 Gewichtsprozent Polyisobutylen-Urethan, Polyisobutylen-Harnstoff oder Polyisobutylen-Urethan/Harnstoff-Copolymer umfasst.

**8.** Verfahren nach Anspruch 5, wobei vor dem Elektrospinnen eine Beschichtungslösung hergestellt wird, die zwischen 2 und 25 Gewichtsprozent Polyisobutylen-Urethan, Polyisobutylen-Harnstoff oder Polyisobutylen-Urethan/-Harnstoff-Copolymer umfasst.

**9.** Verfahren nach Anspruch 5, wobei das Ausbilden der Fasermatrix ein Schmelzblasen des Polyisobutylen-Urethans, Polyisobutylen-Harnstoffs oder Polyisobutylen-Urethan/Harnstoff-Copolymers umfasst.

**10.** Verfahren nach Anspruch 5, wobei das Ausbilden der Fasermatrix das Ausbilden der Fasermatrix auf einem Substrat und ein Ablegen der Fasermatrix zumindest zum Teil über der Elektrode umfasst.

**11.** Verfahren nach Anspruch 5, wobei das Ausbilden der Fasermatrix das Ausbilden der Fasermatrix zumindest zum Teil über der Elektrode umfasst.

**12.** Medizinische elektrische Leitung nach Anspruch 1, wobei die Fasermatrix eine durchschnittliche Fasergröße im Bereich von 10 Nanometern bis 400 Nanometern, eine Porosität von mindestens 40 %, eine durchschnittliche Porengröße im Bereich von 70 Nanometern bis 250 Nanometern und eine Dicke von 10 Nanometern bis 10 Mikrometern aufweist.

**13.** Medizinische elektrische Leitung nach Anspruch 1, wobei die Fasermatrix eine durchschnittliche Fasergröße im Bereich von 400 Nanometern bis 1000 Nanometern, eine Porosität von mindestens 40 %, eine durchschnittliche Porengröße im Bereich von 1 Mikrometer bis 100 Mikrometern und eine Dicke im Bereich von 10 Nanometern bis 10 Mikrometern aufweist.

**14.** Medizinische elektrische Leitung nach Anspruch 1, wobei das weiche Segment zu mindestens 70 Gewichtsprozent mindestens ein Polyisobutylenmakrodiol und/oder -diamin enthält.

## Revendications

**1.** Dérivation électrique médicale comprenant :

un corps de dérivation isolant qui s'étend depuis une région distale jusqu'à une région proximale ;
un conducteur qui est disposé à l'intérieur du corps de dérivation isolant et qui s'étend depuis la région proximale jusqu'à la région distale ;
une électrode qui est disposée sur le corps de dérivation isolant et qui est en contact électrique avec le conducteur ; et
une matrice fibreuse non conductrice qui est disposée au moins partiellement au-dessus de l'électrode,
la matrice fibreuse comprenant un polyisobutylène-uréthane, une polyisobutylène-urée ou un copolymère polyisobutylène-uréthane/polyisobutylène-urée qui comprend de 10 % à 60 % en poids d'un segment dur et le complément en poids d'un segment mou, et dans laquelle la matrice fibreuse comprend une pluralité de fibres alignées de façon aléatoire qui présentent un diamètre de fibre moyen qui s'inscrit à l'intérieur de la plage qui va de 10 nanomètres à 400 nanomètres.

**2.** Dérivation électrique médicale selon la revendication 1, dans laquelle le segment dur inclut un polyisobutylène-uréthane, une polyisobutylène-urée ou un copolymère polyisobutylène-uréthane/polyisobutylène-urée, et dans laquelle le segment mou inclut au moins un polyisobutylène macrodiol ou une diamine.

**3.** Dérivation électrique médicale selon la revendication 1, dans laquelle la matrice fibreuse présente une porosité d'au moins 40 %, une taille de pore moyenne qui s'inscrit à l'intérieur de la plage qui va de 10 nanomètres à 10 micromètres et une épaisseur qui s'inscrit à l'intérieur de la plage qui va de 1 nanomètre à 1 000 micromètres.

**4.** Dérivation électrique médicale selon la revendication 1, dans laquelle la matrice fibreuse présente une porosité d'au moins 40 %, 60 %, 75 % ou 80 %.

**5.** Procédé de formation d'une dérivation électrique médicale qui comporte un corps de dérivation isolant et une électrode qui est disposée sur le corps de dérivation isolant, le procédé comprenant les étapes constituées par :

la formation d'une matrice fibreuse qui comprend au moins un polyisobutylène-uréthane, une polyisobutylène-urée ou un copolymère polyisobutylène-uréthane/polyisobutylène-urée par électrofilage ou extrusion soufflage ; et
la disposition de la matrice fibreuse au moins partiellement au-dessus de l'électrode ;
dans lequel la matrice fibreuse comprend un polyisobutylène-uréthane, une polyisobutylène-urée ou un copolymère polyisobutylène-uréthane/polyisobutylène-urée qui comprend de 10 % à 60 % en poids d'un segment dur et le complément en poids d'un segment mou, et dans lequel la matrice fibreuse comprend une pluralité de fibres alignées de façon aléatoire qui présentent un diamètre de fibre moyen qui s'inscrit à l'intérieur de la plage qui va de 10 nanomètres à 400 nanomètres.

**6.** Procédé selon la revendication 5, dans lequel la formation de la matrice fibreuse comprend l'électrofilage du polyisobutylène-uréthane, de la polyisobutylène-urée ou du copolymère polyisobutylène-uréthane/polyisobutylène-urée.

**7.** Procédé selon la revendication 5, dans lequel, avant l'électrofilage, une solution de revêtement qui comprend entre 1 % et 30 % en poids du polyisobutylène-uréthane, de la polyisobutylène-urée ou du copolymère polyisobutylène-uréthane/polyisobutylène-urée est préparée.

8. Procédé selon la revendication 5, dans lequel, avant l'électrofilage, une solution de revêtement qui comprend entre 2 % et 25 % en poids du polyisobutylène-uréthane, de la polyisobutylène-urée ou du copolymère polyisobutylène-uréthane/polyisobutylène-urée est préparée.

9. Procédé selon la revendication 5, dans lequel la formation de la matrice fibreuse comprend l'extrusion soufflage du polyisobutylène-uréthane, de la polyisobutylène-urée ou du copolymère polyisobutylène-uréthane/polyisobutylène-urée.

10. Procédé selon la revendication 5, dans lequel la formation de la matrice fibreuse comprend la formation de la matrice fibreuse sur un substrat et le dépôt de la matrice fibreuse au moins partiellement au-dessus de l'électrode.

11. Procédé selon la revendication 5, dans lequel la formation de la matrice fibreuse comprend la formation de la matrice fibreuse au moins partiellement au-dessus de l'électrode.

12. Dérivation électrique médicale selon la revendication 1, dans laquelle la matrice fibreuse présente une taille de fibre moyenne qui s'inscrit à l'intérieur de la plage qui va de 10 nanomètres à 400 nanomètres, une porosité d'au moins 40 %, une taille de pore moyenne qui s'inscrit à l'intérieur de la plage qui va de 70 nanomètres à 250 nanomètres et une épaisseur qui s'inscrit à l'intérieur de la plage qui va de 10 nanomètres à 10 micromètres.

13. Dérivation électrique médicale selon la revendication 1, dans laquelle la matrice fibreuse présente une taille de fibre moyenne qui s'inscrit à l'intérieur de la plage qui va de 400 nanomètres à 1 000 nanomètres, une porosité d'au moins 40 %, une taille de pore moyenne qui s'inscrit à l'intérieur de la plage qui va de 1 micromètre à 100 micromètres et une épaisseur qui s'inscrit à l'intérieur de la plage qui va de 10 nanomètres à 10 micromètres.

14. Dérivation électrique médicale selon la revendication 1, dans laquelle le segment mou inclut au moins 70 % en poids d'au moins un polyisobutylène macrodiol et/ou une diamine.

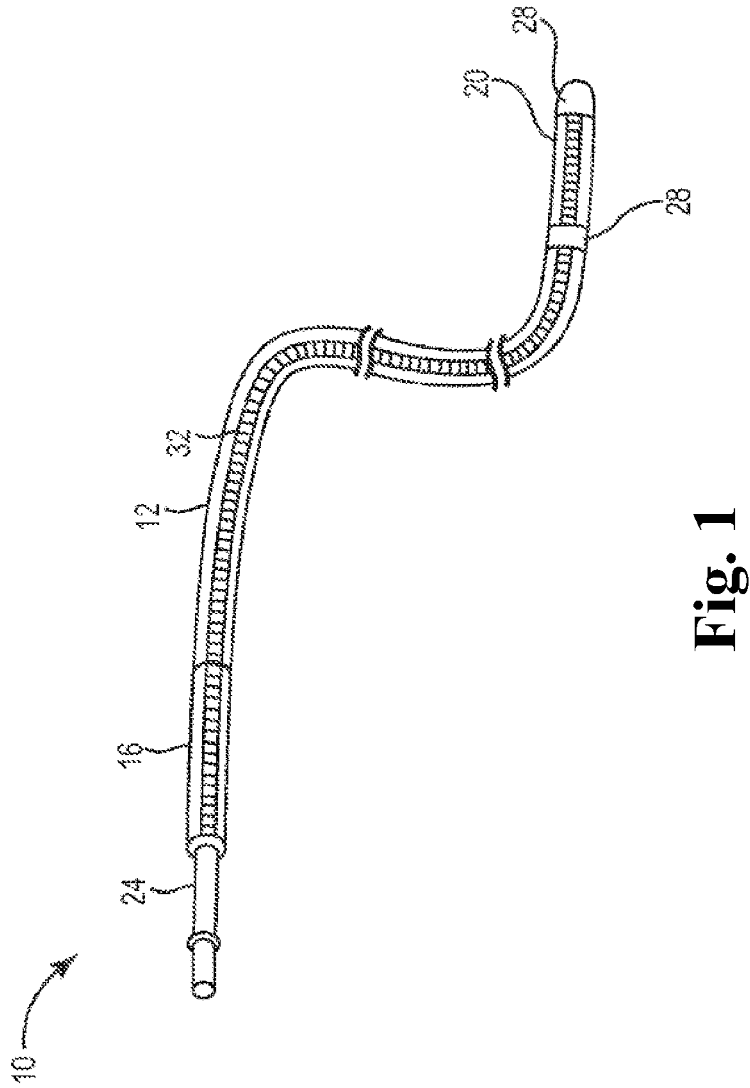


Fig. 1

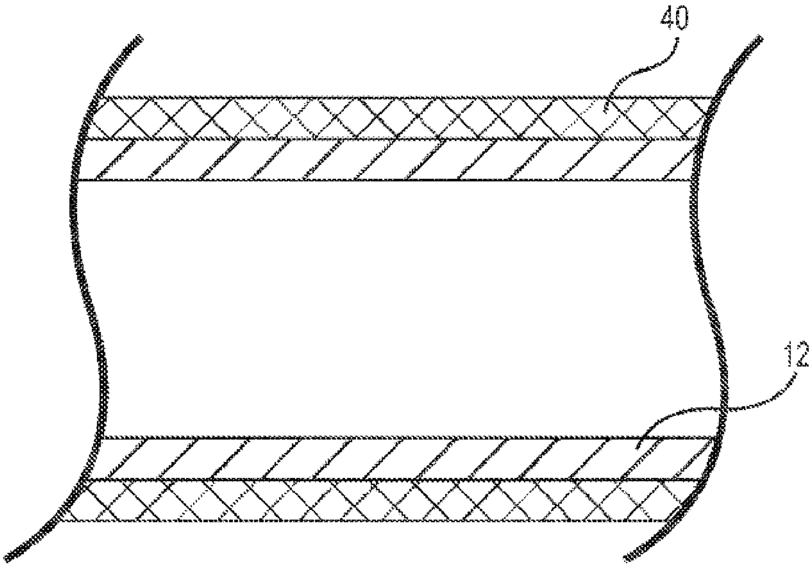


Fig. 2A

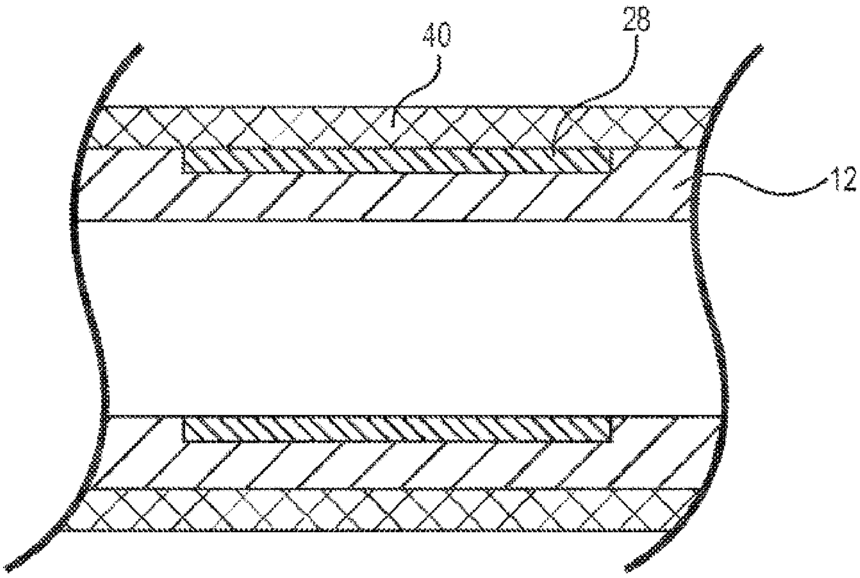


Fig. 2B

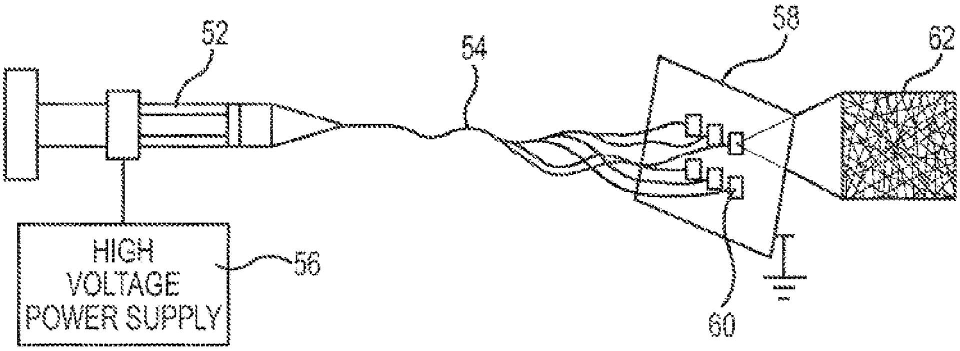
52
54
58
62
HIGH
VOLTAGE
POWER SUPPLY
56
60

Fig. 3

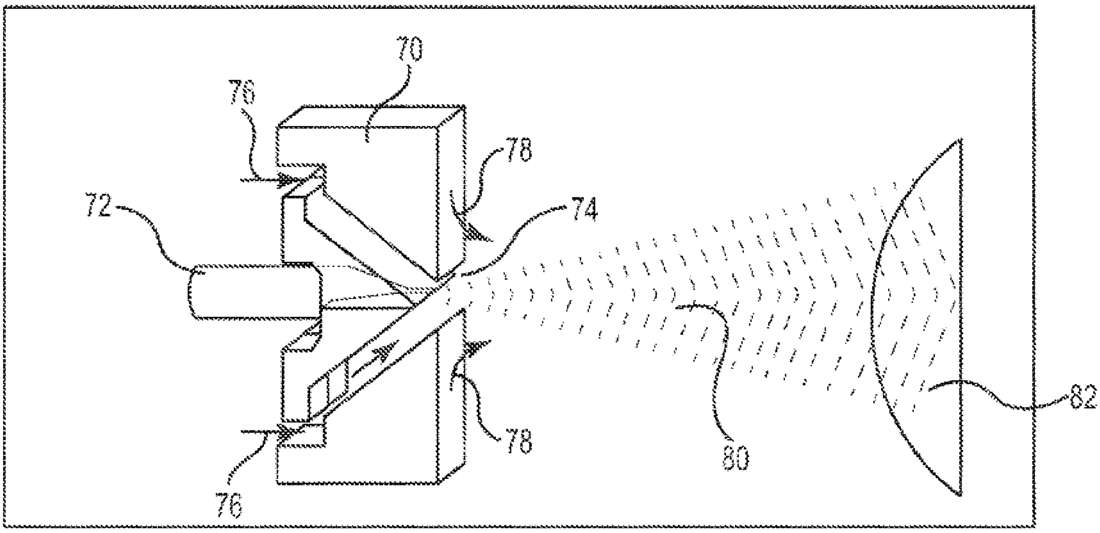
70
76
78
72
74
80
82
78
76

Fig. 4

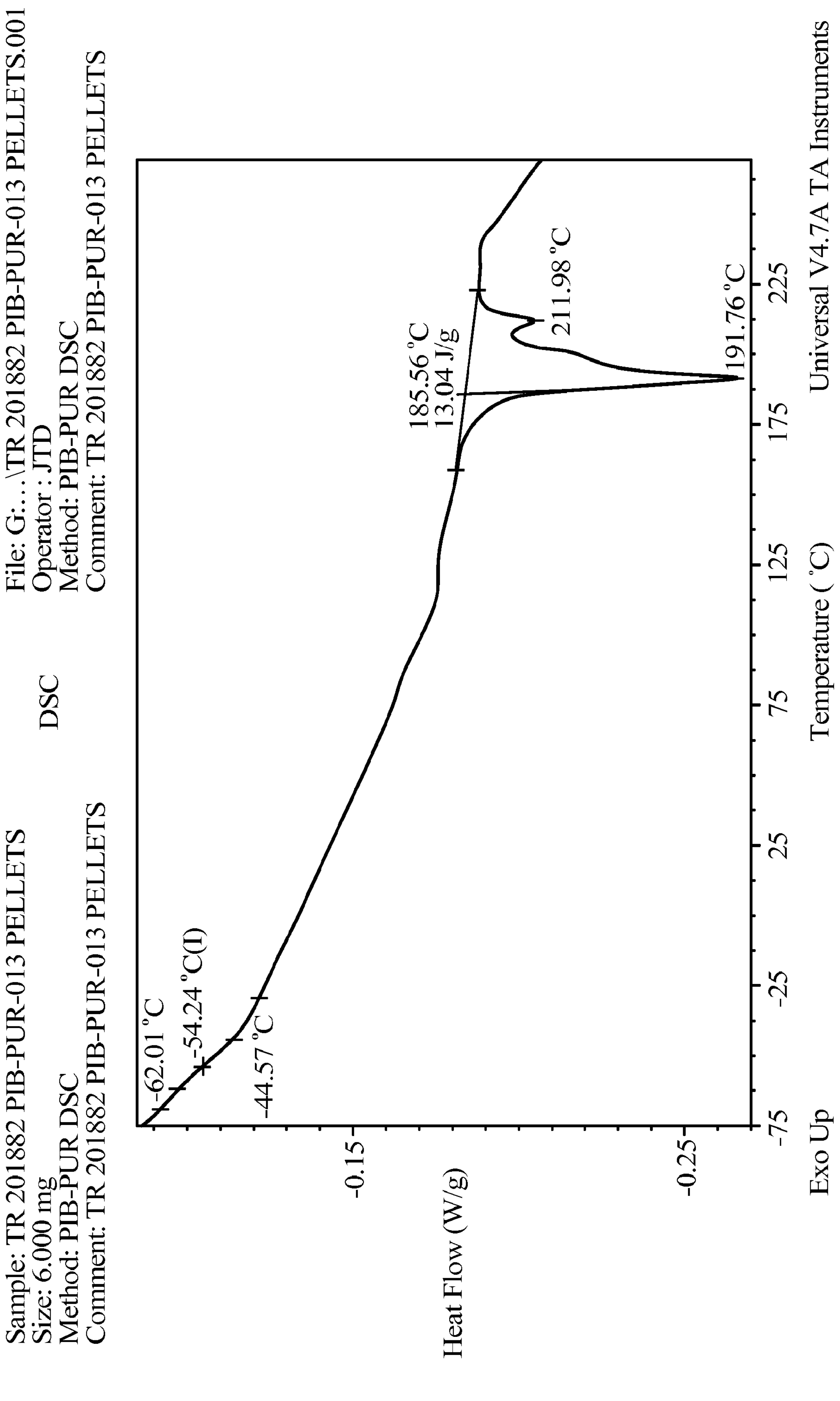
Sample: TR 201882 PIB-PUR-013 PELLETS
Size: 6.000 mg
Method: PIB-PUR DSC
Comment: TR 201882 PIB-PUR-013 PELLETS
DSC
File: G:...\TR 201882 PIB-PUR-013 PELLETS.001
Operator : JTD
Method: PIB-PUR DSC
Comment: TR 201882 PIB-PUR-013 PELLETS
Heat Flow (W/g)
-0.15
-0.25
Exo Up
-62.01 °C
-54.24 °C(I)
-44.57 °C
185.56 °C
13.04 J/g
191.76 °C
211.98 °C
-75
-25
25
75
125
175
225
Temperature (°C)
Universal V4.7A TA Instruments

**Fig. 5**

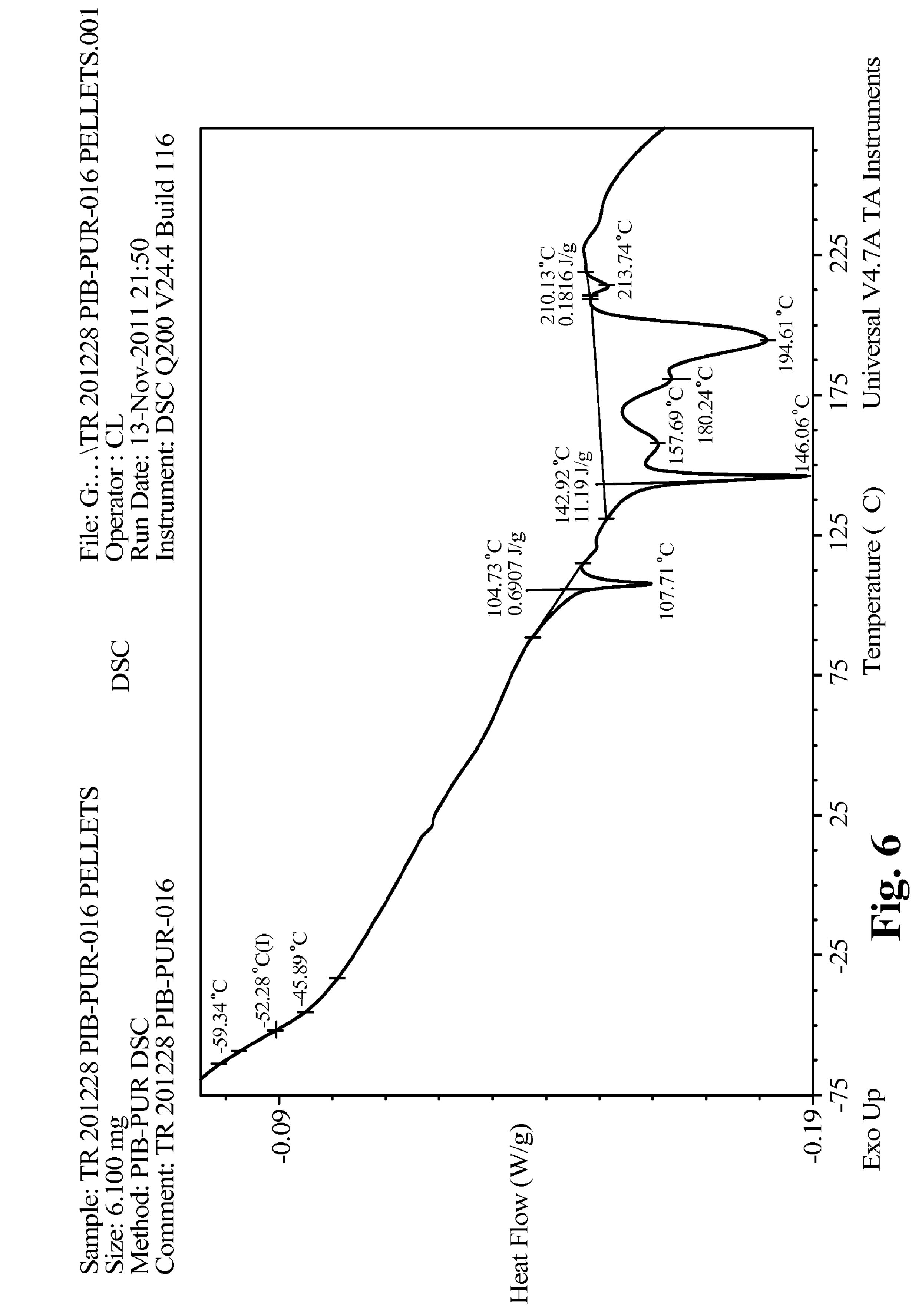

Sample: TR 201228 PIB-PUR-016 PELLETS
Size: 6.100 mg
Method: PIB-PUR DSC
Comment: TR 201228 PIB-PUR-016
DSC
File: G:...\TR 201228 PIB-PUR-016 PELLETS.001
Operator: CL
Run Date: 13-Nov-2011 21:50
Instrument: DSC Q200 V24.4 Build 116
Heat Flow (W/g)
-0.09
-0.19
-59.34°C
-52.28°C(I)
-45.89°C
104.73°C
0.6907 J/g
107.71°C
142.92°C
11.19 J/g
146.06°C
157.69°C
180.24°C
194.61°C
210.13°C
0.1816 J/g
213.74°C
-75
-25
25
75
125
175
225
Exo Up
Temperature (°C)
Universal V4.7A TA Instruments


**Fig. 6**

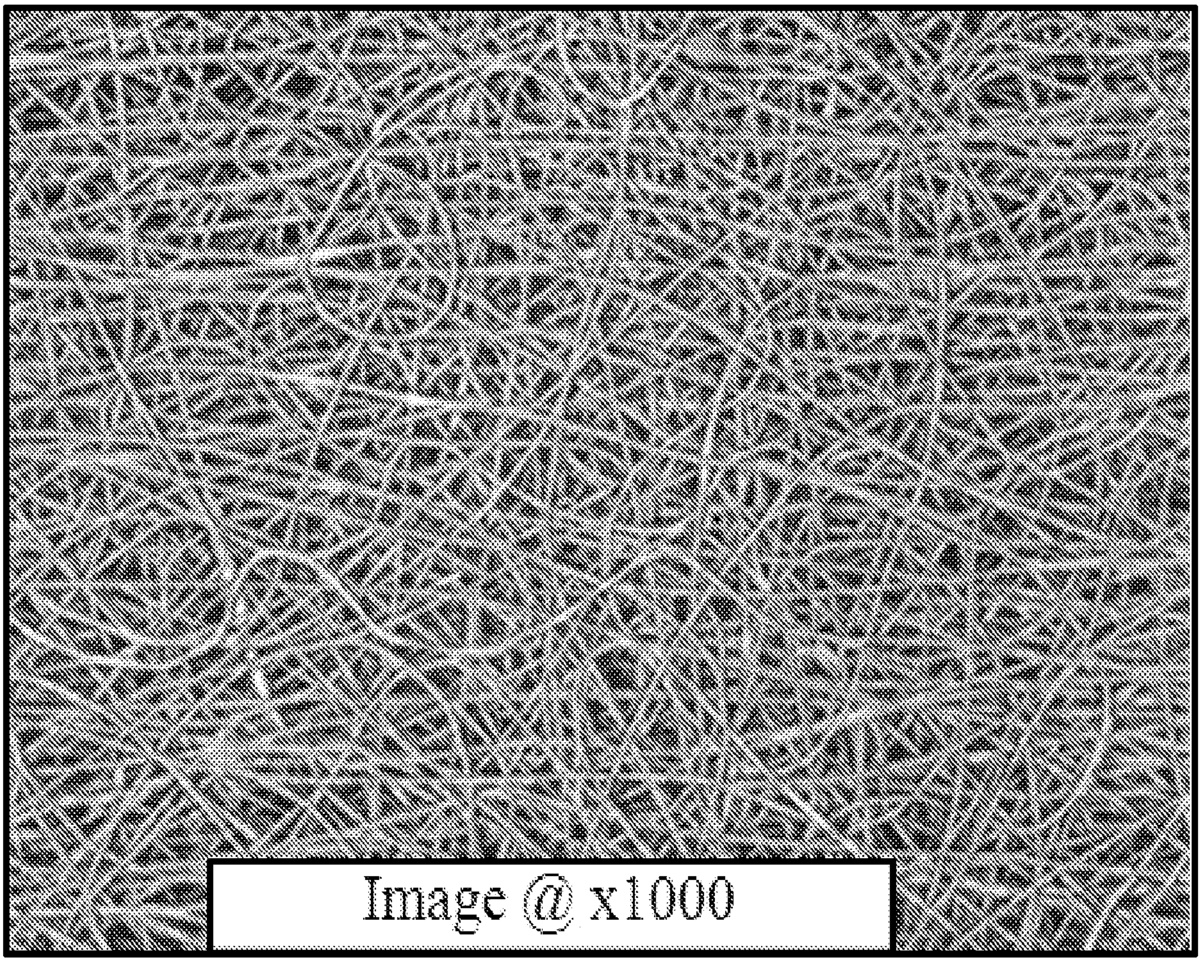


Fig. 7

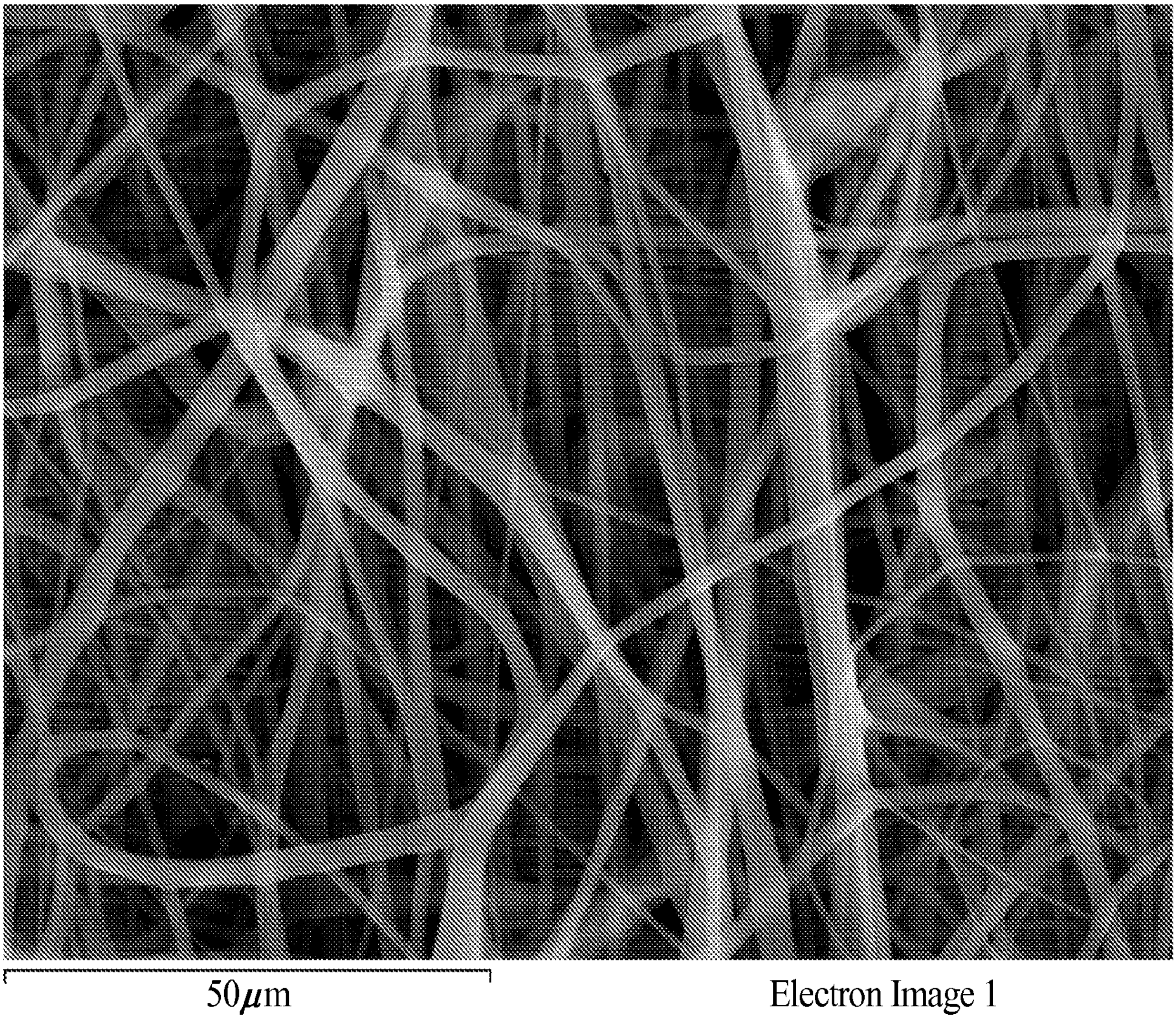


Fig. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20110054581 A1
- US 20070255378 A1
- WO 2008060333 A
- WO 2008066914 A
- US 49248309
- US 87488710


### Non-patent literature cited in the description


- **J.P. KENNEDY et al.** Designed Polymers by Carbocationic Macromolecular Engineering: Theory and Practice. Hanser Publishers, 1991, 191-193
- **JOSEPH P. KENNEDY.** *Journal of Elastomers and Plastics,* 1985, vol. 17, 82-88
- **M. WEIßMÜLLER et al.** Preparation and end-linking of hydroxyl-terminated polystyrene star macromolecules. *Macromolecular Chemistry and Physics,* 1999, vol. 200 (3), 541-551